# EUROPEAN PATENT SPECIFICATION

(11) **EP 4 043 454 B1**
(45) Date of publication and mention of the grant of the patent: **21.05.2025**
(21) Application number: 20875561.1
(22) Date of filing: 07.10.2020
(51) Int. Cl.: C07D 405/14, C07D 409/14, C07F 7/08, C07D 405/04, H10K 85/60, C09K 11/06, H10K 50/11

(54) **HETEROCYCLIC COMPOUND, ORGANIC LIGHT-EMITTING DIODE COMPRISING SAME, COMPOSITION FOR ORGANIC LAYER OF ORGANIC LIGHT-EMITTING DIODE, AND METHOD FOR MANUFACTURING ORGANIC LIGHT-EMITTING DIODE**
HETEROCYCLISCHE VERBINDUNG, ORGANISCHE LICHTEMITTIERENDE DIODE DAMIT, ZUSAMMENSETZUNG FÜR DIE ORGANISCHE SCHICHT EINER ORGANISCHEN LICHTEMITTIERENDEN DIODE UND VERFAHREN ZUR HERSTELLUNG EINER ORGANISCHEN LICHTEMITTIERENDEN DIODE
COMPOSÉ HÉTÉROCYCLIQUE, DIODE ÉLECTROLUMINESCENTE ORGANIQUE LE COMPRENANT, COMPOSITION POUR COUCHE ORGANIQUE DE DIODE ÉLECTROLUMINESCENTE ORGANIQUE, ET PROCÉDÉ DE FABRICATION DE DIODE ÉLECTROLUMINESCENTE ORGANIQUE

(30) Priority: 08.10.2019 KR 20190124521
(43) Date of publication of application: 17.08.2022
(73) Proprietor: LT Materials Co., Ltd., Yongin-si, Gyeonggi-do 17118 (KR)
(72) Inventor: KIM, Su-Yeon, Yongin-si, Gyeonggi-do 17118 (KR); YANG, Seung-Gyu, Yongin-si, Gyeonggi-do 17118 (KR); NO, Young-Seok, Yongin-si, Gyeonggi-do 17118 (KR); PARK, Geon-Yu, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2020/013665
(87) International publication number: WO 2021/071248

(56) References cited:
- JP-A- 2016 149 473
- JP-A- 2016 149 473
- KR-A- 20180 068 869
- KR-A- 20180 068 869
- KR-A- 20190 030 963
- KR-A- 20190 030 963
- KR-A- 20190 079 339
- KR-A- 20190 079 340
- KR-A- 20190 079 340
- KR-A- 20190 079 571

## Description

### [Technical Field]

The present specification relates to a heterocyclic compound, and an organic light emitting device comprising the same.

### [Background Art]

An organic electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a host-dopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

Studies on an organic light emitting device comprising a compound capable of satisfying conditions required for materials usable in an organic light emitting device, for example, satisfying proper energy level, electrochemical stability, thermal stability and the like, and having a chemical structure capable of performing various roles required in an organic light emitting device depending on substituents have been required.

### Prior Art Documents

### Patent Documents

US Patent No. 4,356,429
JP 2016/149473 A discloses an OLED comprising exemplary compounds as follows:

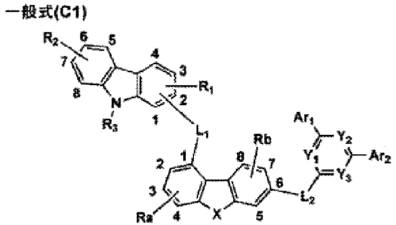

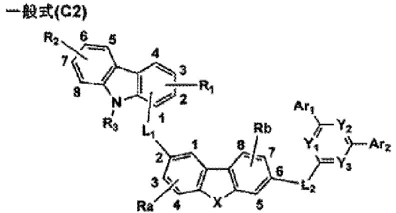

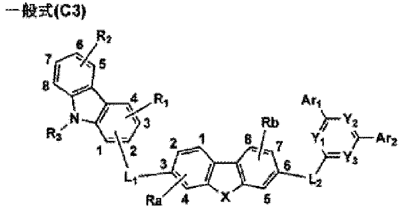

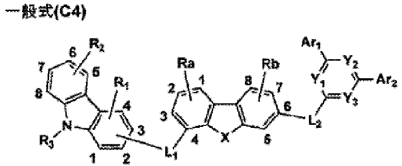

KR 2018/0068869 discloses an OLED comprising a host of formula

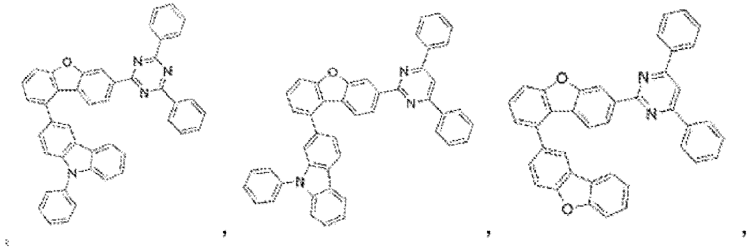

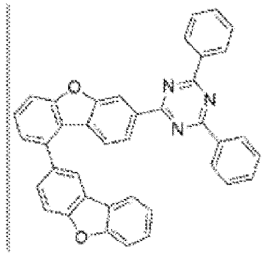

KR 2019/0079340 A discloses an OLED comprising a compound of the formula:

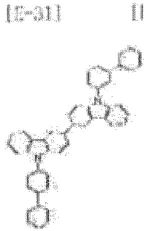

KR 2019/0030963 A discloses an OLED comprising heterocyclic compounds.

### [Disclosure]

### [Technical Problem]

The present application relates to a heterocyclic compound, and an organic light emitting device comprising the same.

### [Technical Solution]

One embodiment of the present application provides a heterocyclic compound represented by any one of the following compounds:

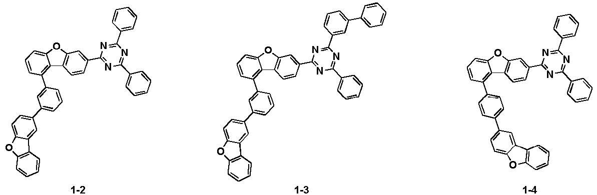

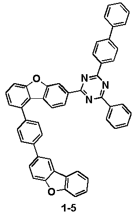

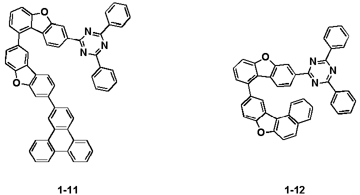

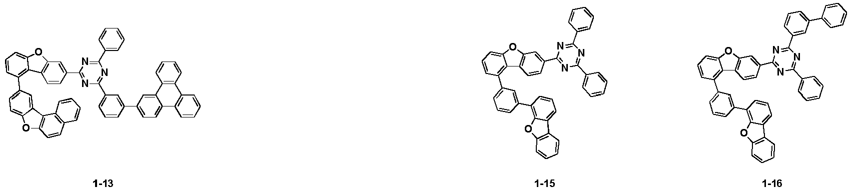

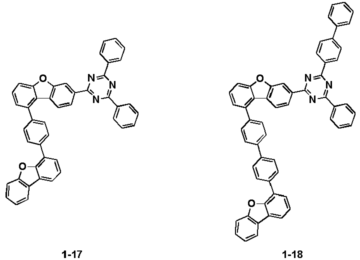

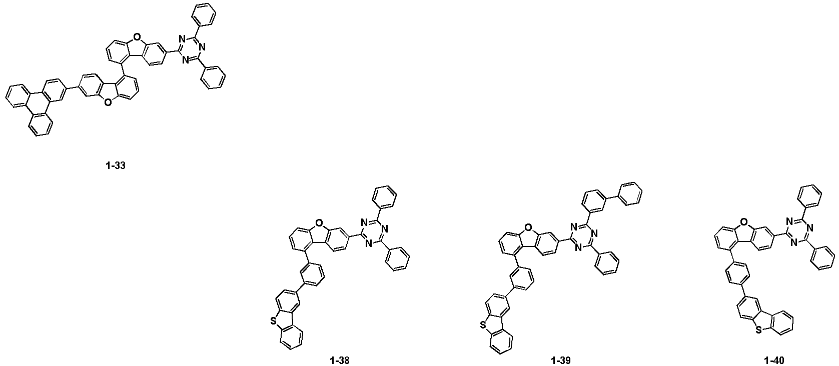

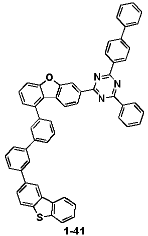

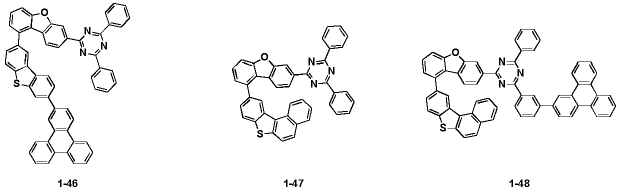

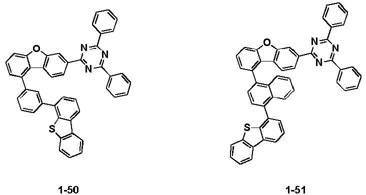

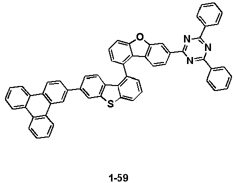

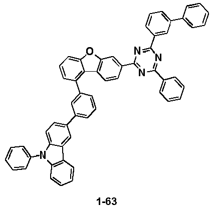

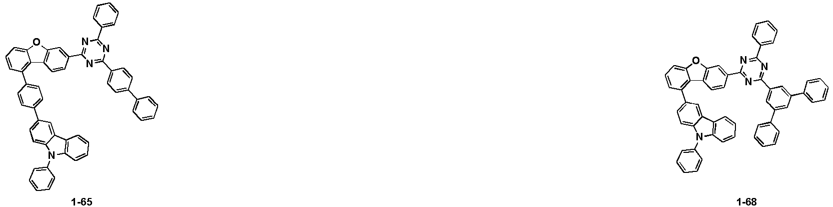

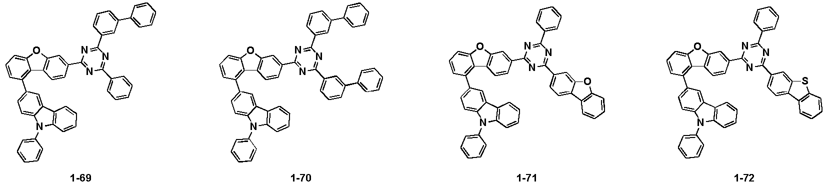

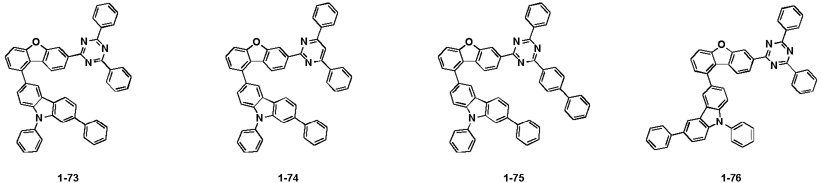

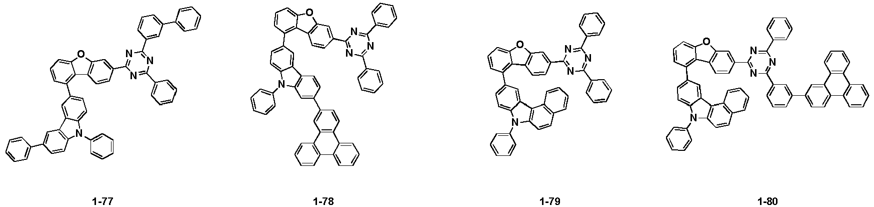

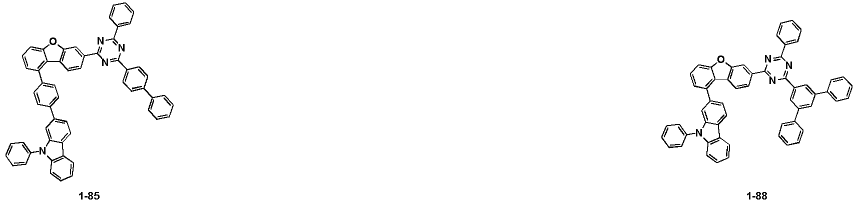

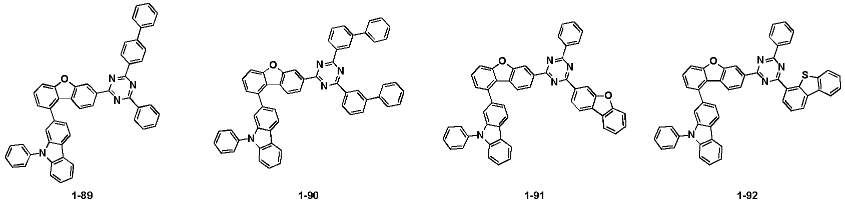

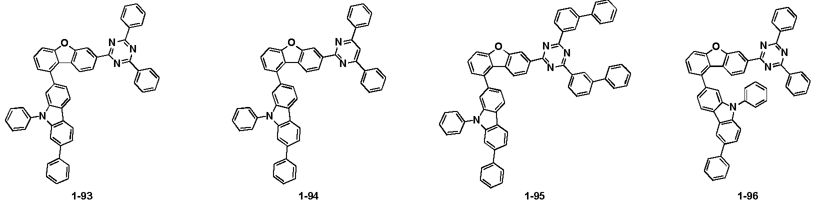

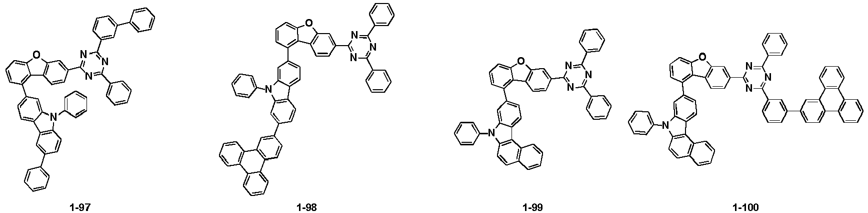

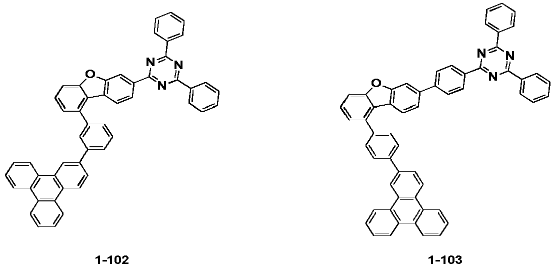

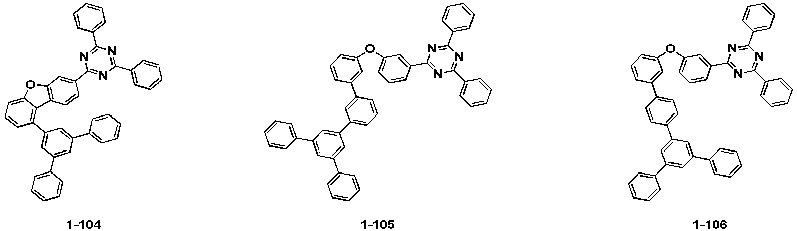

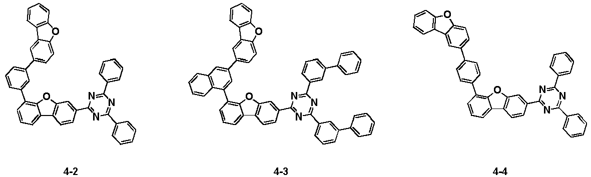

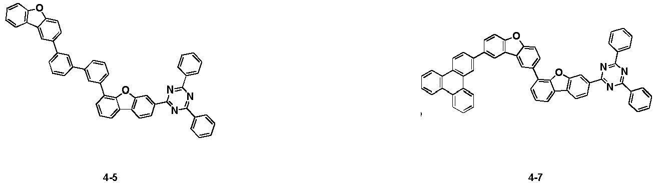

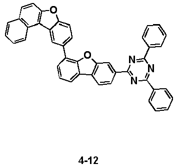

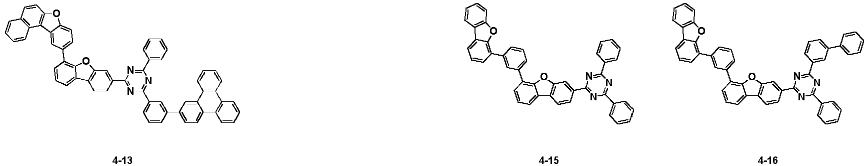

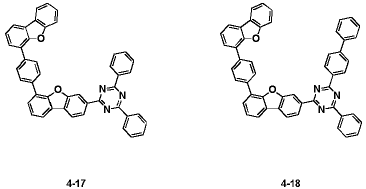

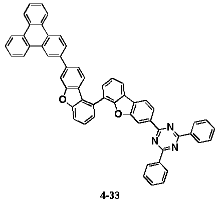

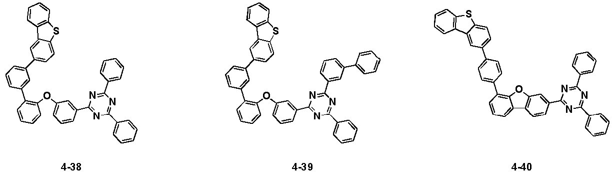

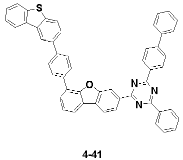

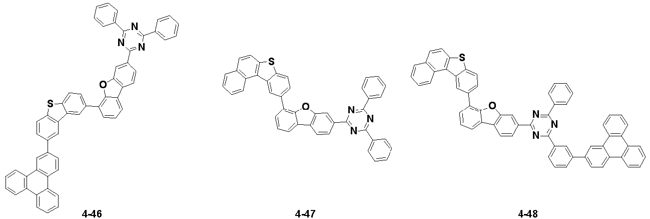

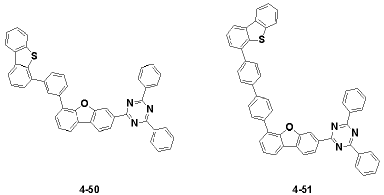

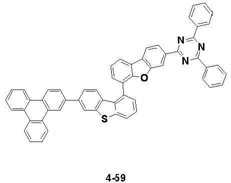

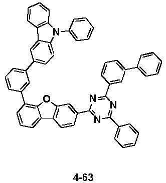

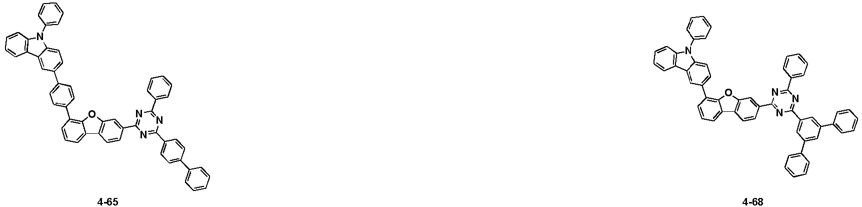

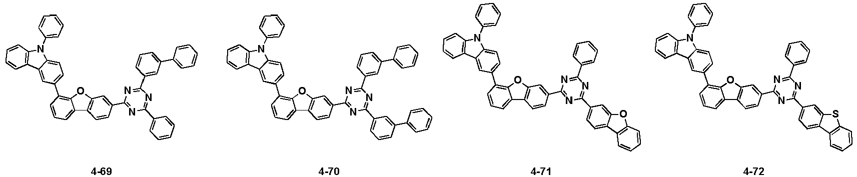

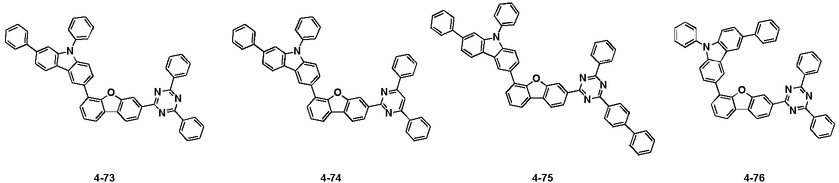

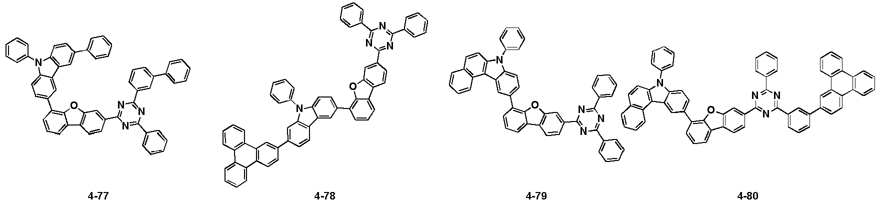

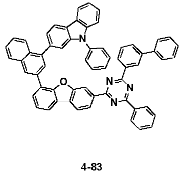

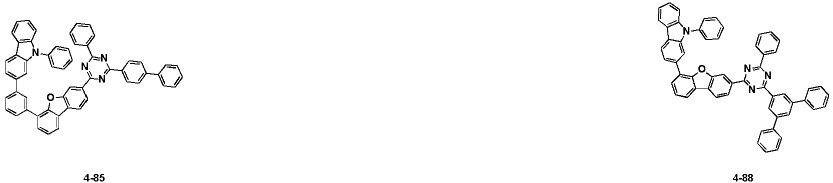

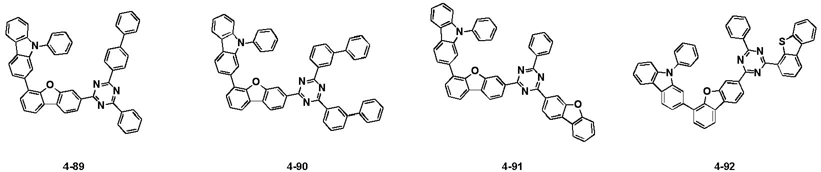

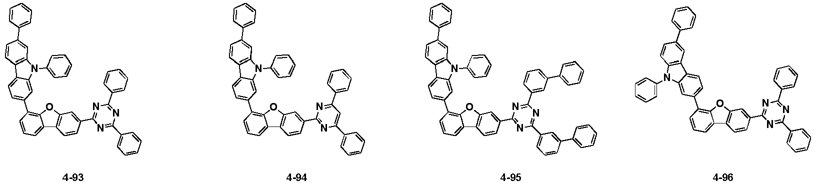

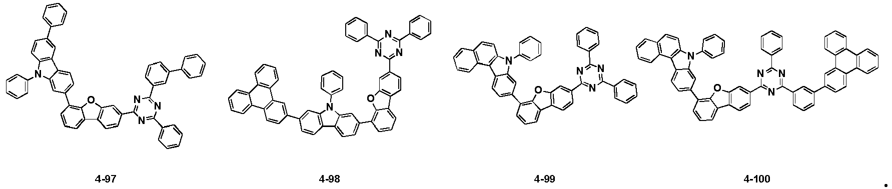

In addition, one embodiment of the present application provides an organic light emitting device comprising a first electrode; a second electrode provided opposite to the first electrode; and one or more organic material layers provided between the first electrode and the second electrode, wherein one or more layers of the organic material layers comprise the heterocyclic compound of the present invention.

Further embodiments are disclosed in the dependent claims.

### [Advantageous Effects]

A compound described in the present specification can be used as a material of an organic material layer of an organic light emitting device. In the organic light emitting device, the compound is capable of performing a role of a hole injection material, a hole transfer material, a light emitting material, an electron transfer material, or an electron injection material. Particularly, the compound can be used as a light emitting material of an organic light emitting device. For example, the compound can be used alone as a light emitting material, or two of the compounds can be used together as a light emitting material, and can be used as a host material of a light emitting layer.

Particularly, by substituting a No. 3 position of one side benzene ring of the dibenzofuran structure with an N-containing ring and substituting another benzene ring not substituted with the N-containing ring in the dibenzofuran ring with a specific substituent, a compound of the invention has a more electron-stable structure by delocalizing LUMO electrons of the N-containing ring side, and provides proper energy level and thermal stability to a device. By using the compounds of the invention, an organic light emitting device with improved lifetime, driving stability and efficiency can be manufactured.

### [Description of Drawings]

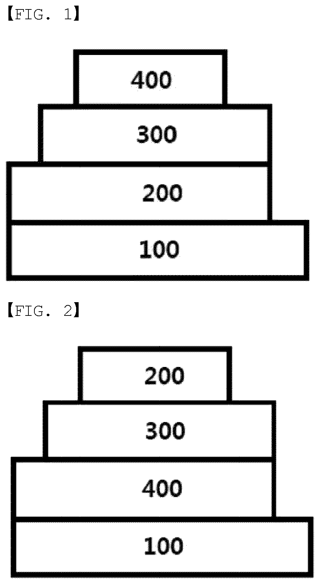

FIG. 1 to FIG. 3 are diagrams each schematically illustrating a lamination structure of an organic light emitting device according to one embodiment of the present application.

### [Reference Numeral]

100: Substrate
200: Anode
300: Organic Material Layer
301: Hole Injection Layer
302: Hole Transfer Layer
303: Light Emitting Layer
304: Hole Blocking Layer
305: Electron Transfer Layer
306: Electron Injection Layer
400: Cathode

### [Mode for Disclosure]

Hereinafter, the present application will be described in detail.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0% or a hydrogen content being 100%. In other words, an expression of "substituent X is hydrogen" does not exclude deuterium such as a hydrogen content being 100% or a deuterium content being 0%, and therefore, may mean a state in which hydrogen and deuterium are mixed.

In one embodiment of the present application, deuterium is one of isotopes of hydrogen, is an element having deuteron formed with one proton and one neutron as a nucleus, and may be expressed as hydrogen-2, and the elemental symbol may also be written as D or 2H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 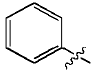 means that the total number of substituents that the phenyl group may have is 5 (T1 in the formula), and the number of deuterium among these is 1 (T2 in the formula). In other words, having a deuterium content of 20% in a phenyl group may be represented by the following structural formulae.

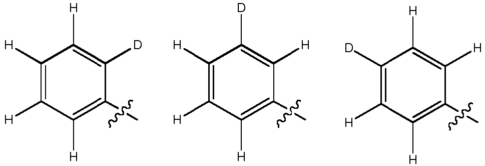

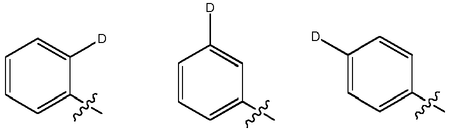

In addition, in one embodiment of the present application, "a phenyl group having a deuterium content of 0%" may mean a phenyl group that does not comprise a deuterium atom, that is, a phenyl group that has 5 hydrogen atoms.

By introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of the compound of the invention, the energy band gap may be finely controlled, and meanwhile, properties at interfaces between organic materials are enhanced, and material applications may become diverse.

In addition, one embodiment of the present application provides an organic light emitting device comprising a first electrode; a second electrode provided opposite to the first electrode; and one or more organic material layers provided between the first electrode and the second electrode, wherein one or more layers of the organic material layers comprise the heterocyclic compound according to the invention.

In one embodiment of the present application, the first electrode may be an anode, and the second electrode may be a cathode.

In another embodiment, the first electrode may be a cathode, and the second electrode may be an anode.

In one embodiment of the present application, the organic light emitting device may be a blue organic light emitting device, and the heterocyclic compound according to the invention may be used as a material of the blue organic light emitting device. For example, the heterocyclic compound according to the invention may be included in a host material of a blue light emitting layer of the blue organic light emitting device.

In one embodiment of the present application, the organic light emitting device may be a green organic light emitting device, and the heterocyclic compound according to the invention may be used as a material of the green organic light emitting device. For example, the heterocyclic compound according to the inventionmay be included in a host material of a green light emitting layer of the green organic light emitting device.

In one embodiment of the present application, the organic light emitting device may be a red organic light emitting device, and the heterocyclic compound according to the invention may be used as a material of the red organic light emitting device. For example, the heterocyclic compound according to the invention may be included in a host material of a red light emitting layer of the red organic light emitting device.

The organic light emitting device of the present disclosure may be manufactured using common organic light emitting device manufacturing methods and materials except that one or more of the organic material layers are formed using the heterocyclic compound described above.

The heterocyclic compound may be formed into an organic material layer through a solution coating method as well as a vacuum deposition method when manufacturing the organic light emitting device. Herein, the solution coating method means spin coating, dip coating, inkjet printing, screen printing, a spray method, roll coating and the like, but is not limited thereto.

The organic material layer of the organic light emitting device of the present disclosure may be formed in a single layer structure, but may be formed in a multilayer structure in which two or more organic material layers are laminated. For example, the organic light emitting device of the present disclosure may have a structure comprising a hole injection layer, a hole transfer layer, a light emitting layer, an electron transfer layer, an electron injection layer and the like as the organic material layer. However, the structure of the organic light emitting device is not limited thereto, and may comprise a smaller number of organic material layers.

In the organic light emitting device of the present disclosure, the organic material layer may comprise a light emitting layer, and the light emitting layer may comprise the heterocyclic compound.

In another organic light emitting device, the organic material layer comprises a light emitting layer, the light emitting layer comprises a host material, and the host material may comprise the heterocyclic compound.

As another example, the organic material layer comprising the heterocyclic compound comprises the heterocyclic compound of the invention as a host, and an iridium-based dopant may be used therewith.

In the organic light emitting device of the present disclosure, the organic material layer comprises an electron injection layer or an electron transfer layer, and the electron transfer layer or the electron injection layer may comprise the heterocyclic compound.

In another organic light emitting device, the organic material layer comprises an electron blocking layer or a hole blocking layer, and the electron blocking layer or the hole blocking layer may comprise the heterocyclic compound.

The organic light emitting device of the present disclosure may further comprise one, two or more layers selected from the group consisting of a light emitting layer, a hole injection layer, a hole transfer layer, an electron injection layer, an electron transfer layer, an electron blocking layer and a hole blocking layer.

FIG 1 to FIG. 3 illustrate a lamination order of electrodes and organic material layers of an organic light emitting device according to one embodiment of the present application. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

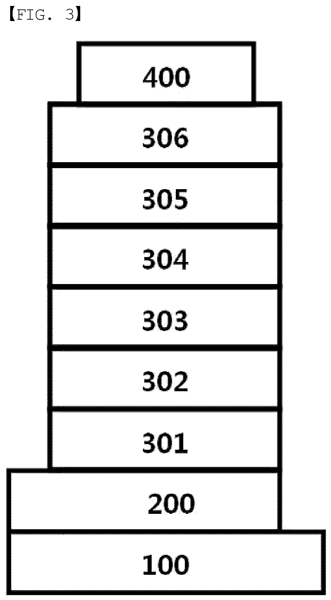

FIG. 3 illustrates a case of the organic material layer being a multilayer. The organic light emitting device according to FIG. 3 comprises a hole injection layer (301), a hole transfer layer (302), a light emitting layer (303), a hole blocking layer (304), an electron transfer layer (305) and an electron injection layer (306). However, the scope of the present application is not limited to such a lamination structure, and as necessary, layers other than the light emitting layer may not be included, and other necessary functional layers may be further added.

The organic material layer comprising the compound of the invention may further comprise other materials as necessary.

In the organic light emitting device according to one embodiment of the present application, the organic material layer may further comprise a heterocyclic compound of the following Chemical Formula 2.

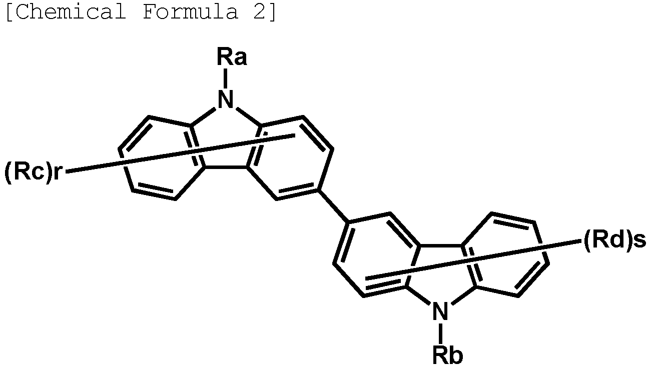

In Chemical Formula 2,
Ra and Rb are the same as or different from each other, and each independently a substituted or unsubstituted C6 to C60 aryl group; or a substituted or unsubstituted C2 to C60 heteroaryl group,
Rc and Rd are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; and a substituted or unsubstituted amine group, and
r and s are an integer of 0 to 7.

Effects of more superior efficiency and lifetime are obtained when comprising the compound of the invention and the compound of Chemical Formula 2 at the same time in the organic material layer of the organic light emitting device. Such results may lead to a forecast that an exciplex phenomenon occurs when comprising the two compounds at the same time.

The exciplex phenomenon is a phenomenon of releasing energy having sizes of a donor (p-host) HOMO level and an acceptor (n-host) LUMO level due to electron exchanges between two molecules. When the exciplex phenomenon occurs between two molecules, reverse intersystem crossing (RISC) occurs, and as a result, internal quantum efficiency of fluorescence may increase up to 100%. When a donor (p-host) having a favorable hole transfer ability and an acceptor (n-host) having a favorable electron transfer ability are used as a host of a light emitting layer, holes are injected to the p-host and electrons are injected to the n-host, and therefore, a driving voltage may be lowered, which resultantly helps with enhancement in the lifetime.

In one embodiment of the present application, Rc and Rd may be hydrogen.

In one embodiment of the present application, Ra and Rb of Chemical Formula 2 are the same as or different from each other, and may be each independently a substituted or unsubstituted C6 to C60 aryl group.

In another embodiment, Ra and Rb of Chemical Formula 2 are the same as or different from each other, and may be each independently a substituted or unsubstituted C6 to C40 aryl group.

In another embodiment, Ra and Rb of Chemical Formula 2 are the same as or different from each other, and may be each independently a C6 to C40 aryl group unsubstituted or substituted with one or more substituents selected from the group consisting of a C1 to C40 alkyl group, a C6 to C40 aryl group, -CN and -SiR201R202R203.

In another embodiment, Ra and Rb of Chemical Formula 2 are the same as or different from each other, and may be each independently a phenyl group unsubstituted or substituted with a phenyl group, -CN or -SiR201R202R203; a biphenyl group unsubstituted or substituted with a phenyl group; a naphthyl group; a fluorene group unsubstituted or substituted with a methyl group or a phenyl group; a spirobifluorene group; or a triphenylene group.

In one embodiment of the present application, R201, R202 and R203 of Chemical Formula 2 may be a C6 to C60 aryl group.

In another embodiment, R201, R202 and R203 of Chemical Formula 2 may be a C6 to C40 aryl group.

In one embodiment of the present application, R201, R202 and R203 of Chemical Formula 2 may be a phenyl group.

In one embodiment of the present application, Chemical Formula 2 may be represented by any one of the following compounds, but is not limited thereto.

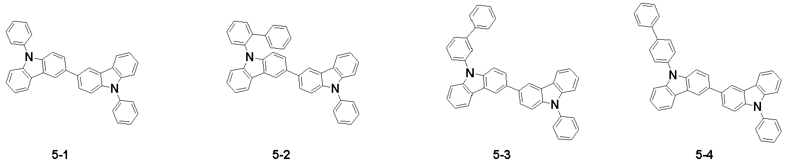

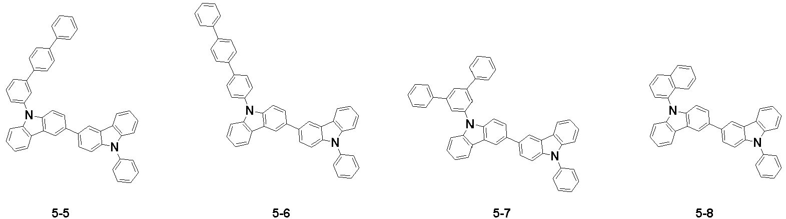

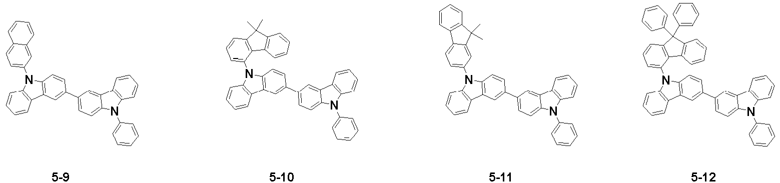

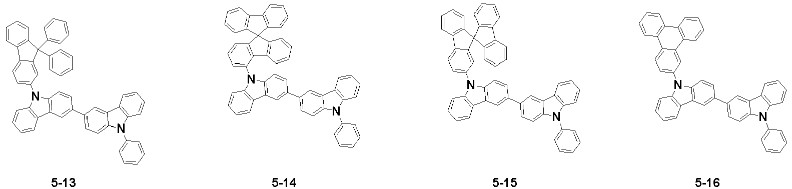

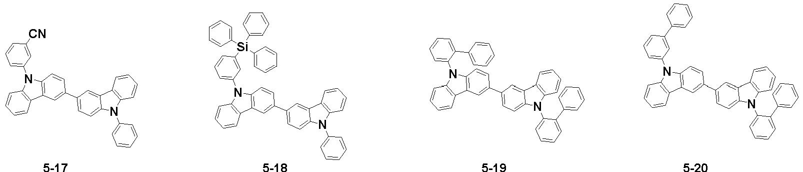

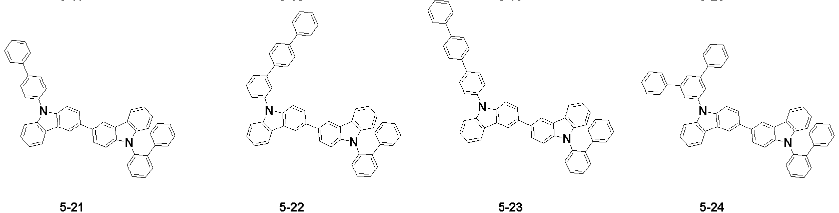

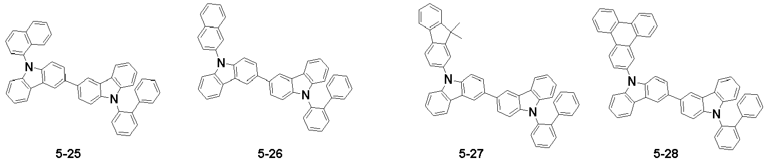

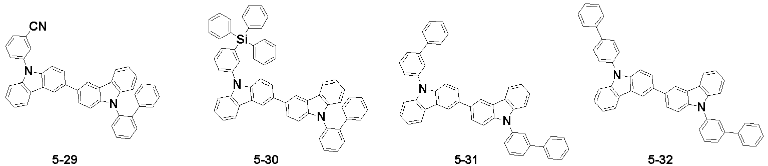

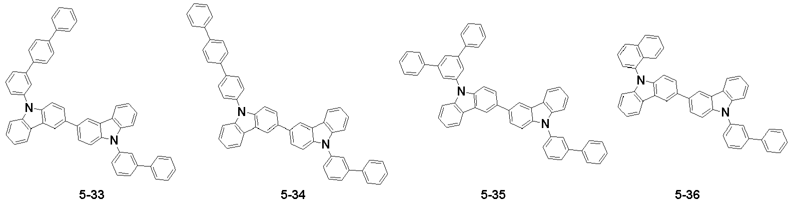

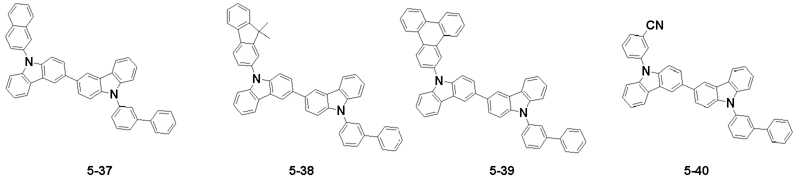

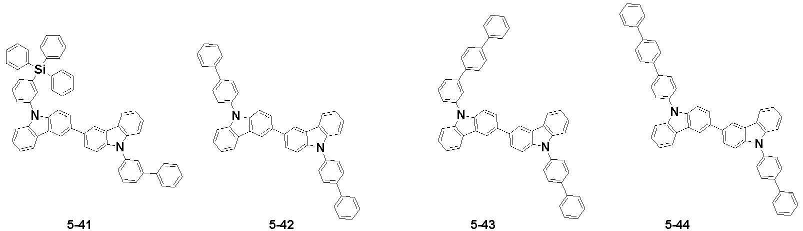

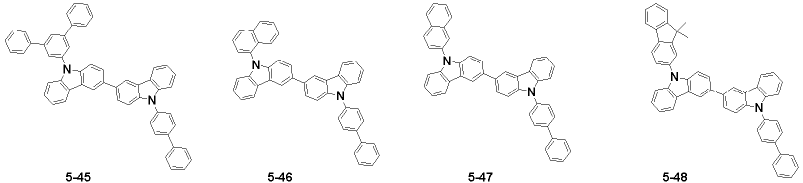

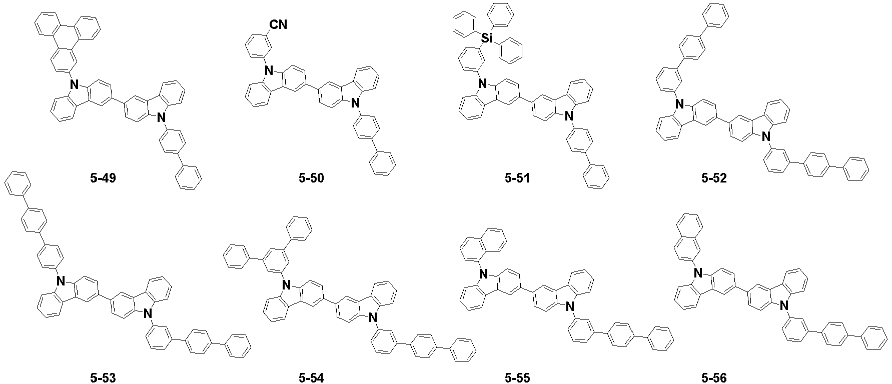

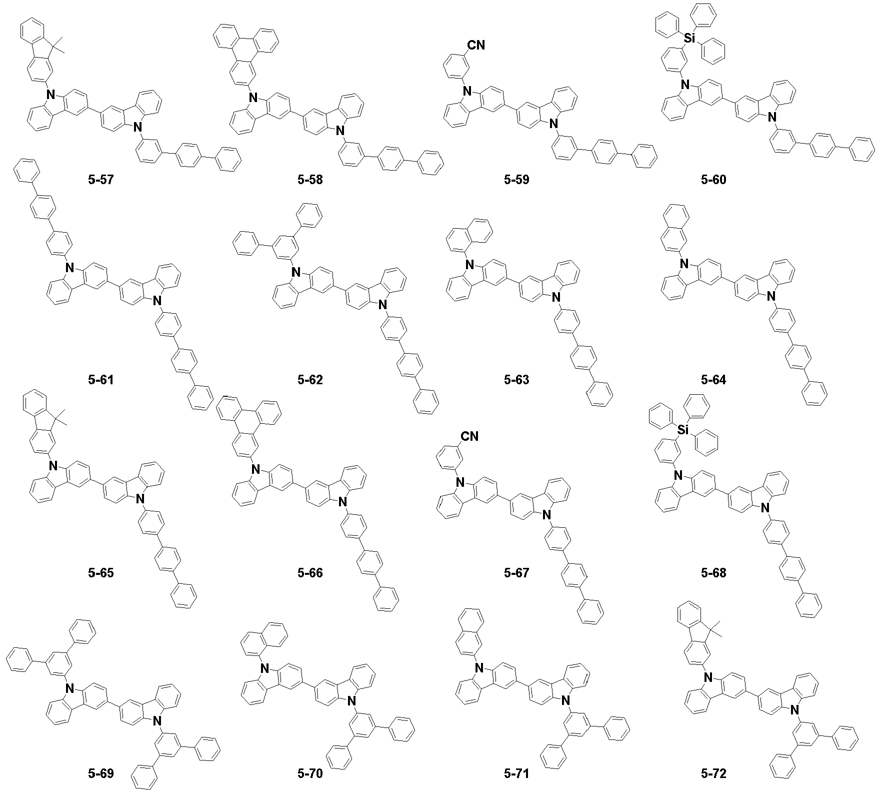

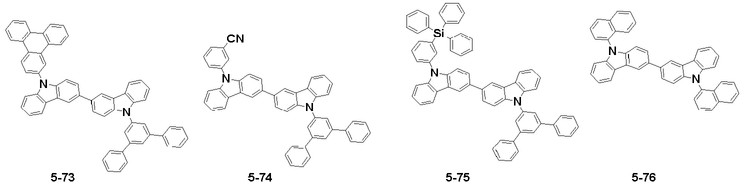

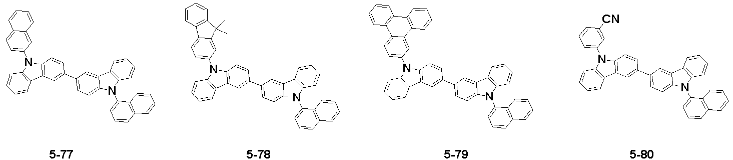

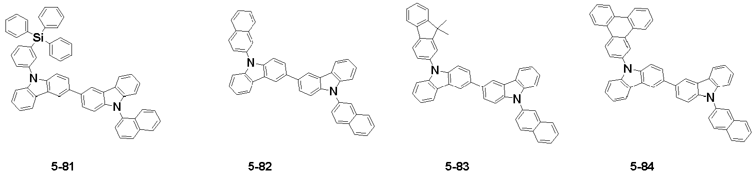

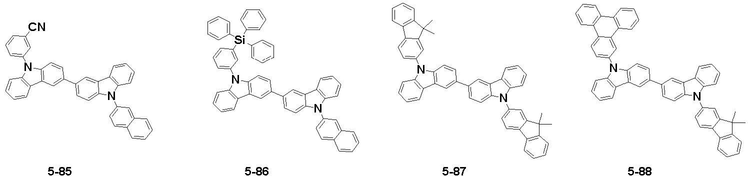

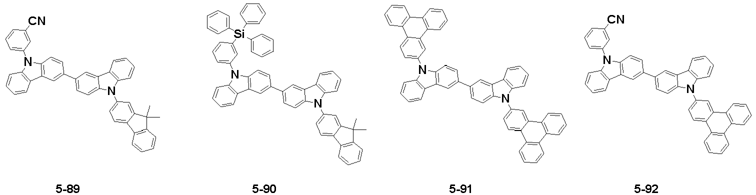

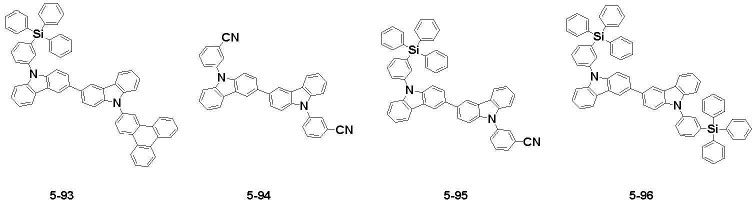

In the organic light emitting device according to one embodiment of the present application, the compound of Chemical Formula 2 may be included in a light emitting layer of the organic material layer.

In the organic light emitting device according to one embodiment of the present application, the compound of Chemical Formula 2 may be included in a light emitting layer of the organic material layer, and may be specifically used as a host material of the light emitting layer.

In one embodiment of the present application, the host material of the light emitting layer of the organic light emitting device may comprise the heterocyclic compound of the invention and the heterocyclic compound of Chemical Formula 2 at the same time.

In the organic light emitting device according to one embodiment of the present application, materials other than the compound of the inventionare illustrated below, however, these are for illustrative purposes only and not for limiting the scope of the present application, and may be replaced by materials known in the art.

As the anode material, materials having relatively large work function may be used, and transparent conductive oxides, metals, conductive polymers or the like may be used. Specific examples of the anode material comprise metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy) thiophene] (PEDOT), polypyrrole and polyaniline, and the like, but are not limited thereto.

As the cathode material, materials having relatively small work function may be used, and metals, metal oxides, conductive polymers or the like may be used. Specific examples of the cathode material comprise metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris (4-carbazoyl-9-ylphenyl) amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrene-sulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used, and as necessary, two or more light emitting materials may be mixed and used. Herein, two or more light emitting materials may be used by being deposited as individual sources of supply or by being premixed and deposited as one source of supply. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

When mixing light emitting material hosts, same series hosts may be mixed, or different series hosts may be mixed. For example, any two or more types of materials among n-type host materials or p-type host materials may be selected and used as a host material of a light emitting layer.

The organic light emitting device according to one embodiment of the present application may be a top-emission type, a bottom-emission type or a dual-emission type depending on the materials used.

The heterocyclic compound according to one embodiment of the present application may also be used in an organic electronic device comprising an organic solar cell, an organic photo conductor, an organic transistor and the like under a similar principle used in the organic light emitting device.

Hereinafter, the present specification will be described in more detail with reference to examples, however, these are for illustrative purposes only, and the scope of the present application is not limited thereto.

### <Preparation Example 1> Preparation of Compound 1-1 (not according to the invention)

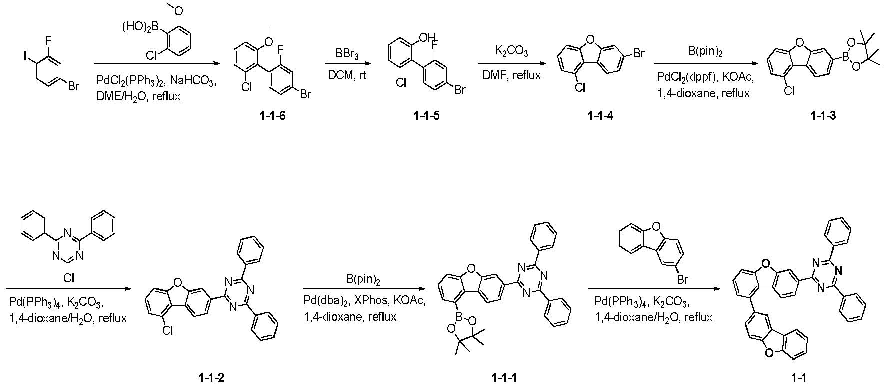

### 1) Preparation of Compound 1-1-6

After dissolving 4-bromo-2-fluoro-1-iodobenzene (200.0 g, 664.7 mM), (2-chloro-6-methoxyphenyl)boronic acid (148.7 g, 794.6 mM), Pd(PPh)₄ (38.4 g, 33.2 mM) and K₂CO₃ (183.7 g, 1329.4 mM) in 1,4-dioxane/H₂O (1 L/200 mL), the result was refluxed for 24 hours. After the reaction was completed, the result was extracted by introducing distilled water and dichloromethane (DCM) thereto at room temperature. The organic layer was dried with MgSO₄, and then the solvent was removed using a rotary evaporator. The reaction material was purified by column chromatography (DCM:Hex=1:3) to obtain target Compound 1-1-6 (178 g, 85%).

### 2) Preparation of Compound 1-1-5

After dissolving Compound 1-1-6 (178 g, 564.1 mM) and BBr₃ (107 mL, 1128.2 mM) in DCM (800 mL), the result was refluxed for 1 hour. After the reaction was completed, the result was extracted by introducing distilled water and DCM thereto at room temperature. The organic layer was dried with MgSO₄, and then the solvent was removed using a rotary evaporator. The reaction material was purified by column chromatography (DCM:Hex=1:1) to obtain target Compound 1-1-5 (153.1 g, 90%).

### 3) Preparation of Compound 1-1-4

After dissolving Compound 1-1-5 (153 g, 507.4 mM) and K₂CO₃ (140.3 g, 1014.8 mM) in dimethylformamide (DMF) (800 mL), the result was refluxed for 4 hours. After the reaction was completed, the result was extracted by introducing distilled water and DCM thereto at room temperature. The organic layer was dried with MgSO₄, and then the solvent was removed using a rotary evaporator. The reaction material was purified by column chromatography (DCM:Hex=1:9), and recrystallized with methanol to obtain target Compound 1-1-4 (88.5 g, 62%).

### 4) Preparation of Compound 1-1-3

After dissolving Compound 1-1-4 (88.5 g, 314.4 mM), bis(pinacolato)diboron (159.7 g, 628.8 mM), PdCl₂(dppf) (23.0 g, 31.4 mM) and KOAc (92.6 g, 943.2 mM) in 1,4-dioxane (500 mL), the result was refluxed for 24 hours. After the reaction was completed, the result was extracted by introducing distilled water and DCM thereto at room temperature. The organic layer was dried with MgSO₄, and then the solvent was removed using a rotary evaporator. The reaction material was purified by column chromatography (DCM:Hex=1:5) to obtain target Compound 1-1-3 (85.7 g, 83%).

### 5) Preparation of Compound 1-1-2

After dissolving Compound 1-1-3 (85.0 g, 258.7 mM), 2-chloro-4,6-diphenyl-1,3,5-triazine (69.3 g, 258.7 mM), Pd(PPh)₄ (14.9 g, 12.9 mM) and K₂CO₃ (71.5 g, 517.4 mM) in 1,4-dioxane/H₂O (1000/200 mL), the result was refluxed for 24 hours. After the reaction was completed, the result was extracted by introducing distilled water and DCM thereto at room temperature. The organic layer was dried with MgSO₄, and then the solvent was removed using a rotary evaporator. The reaction material was purified by column chromatography (DCM:Hex=1:4), and recrystallized with methanol to obtain target Compound 1-1-2 (79.7 g, 71%).

### 6) Preparation of Compound 1-1-1

After dissolving Compound 1-1-2 (79.0 g, 182.1 mM), bis(pinacolato)diboron (92.5 g, 364.2 mM), Pd(dba)₂ (10.5 g, 18.2 mM), XPhos (17.4 g, 36.4 mM) and KOAc (53.6 g, 546.3 mM) in 1,4-dioxane (800 mL), the result was refluxed for 24 hours. After the reaction was completed, the result was extracted by introducing distilled water and DCM thereto at room temperature. The organic layer was dried with MgSO₄, and then the solvent was removed using a rotary evaporator. The reaction material was purified by column chromatography (DCM:Hex=1:5) to obtain target Compound 1-1-1 (84.2 g, 88%).

### 7) Preparation of Compound 1-1

After dissolving Compound 1-1-1 (15.0 g, 28.5 mM), 2-bromodibenzo[b,d]furan (7.8 g, 31.4 mM), Pd(PPh)₄ (1.6 g, 1.4 mM) and K₂CO₃ (7.9 g, 57.0 mM) in 1,4-dioxane/H₂O (200/40 mL), the result was refluxed for 24 hours. After the reaction was completed, the result was extracted by introducing distilled water and DCM thereto at room temperature. The organic layer was dried with MgSO₄, and then the solvent was removed using a rotary evaporator. The reaction material was purified by column chromatography (DCM:Hex=1:3), and recrystallized with methanol to obtain target Compound 1-1 (13.2 g, 82%).

Target Compound A was synthesized in the same manner as in Preparation Example 1 except that Intermediate A of the following Table 1 was used instead of 2-chloro-4,6-diphenyl-1,3,5-triazine, and Intermediate B of the following Table 1 was used instead of 2-bromodibenzo[b,d]furan.

**[Table 1](compounds 1-14, 1-37, 1-49, 1-61 and 1-81 not according to the invention)**

| Compou nd No. | Intermediate A | Intermediate B | Target Compound A | Yield |
|---|---|---|---|---|
| 1-2 | 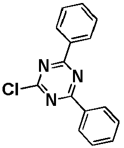 | 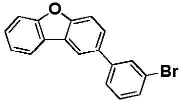 | 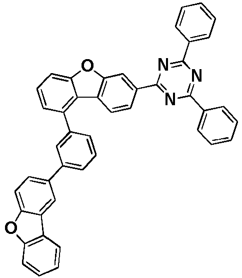 | 20% |
| 1-14 | | 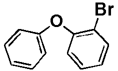 | 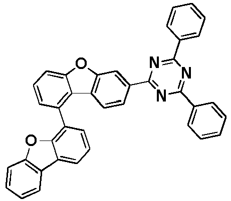 | 26% |
| 1-18 | | 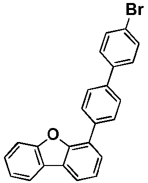 | 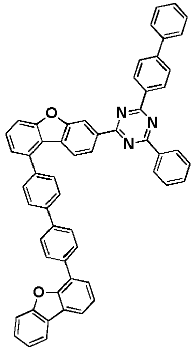 | 22% |
| 1-37 | | 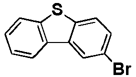 | 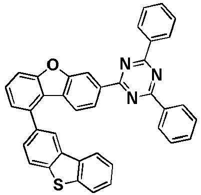 | 23% |
| 1-38 | | 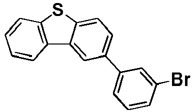 | 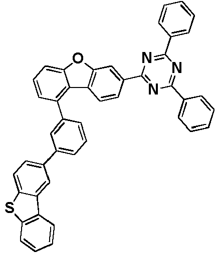 | 25% |
| 1-49 | | 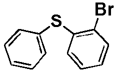 | 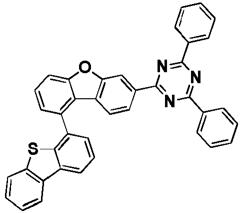 | 24% |
| 1-50 | | 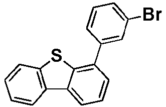 | 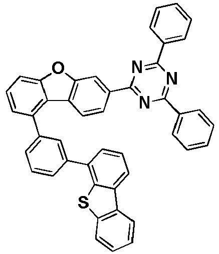 | 22% |
| 1-61 | | 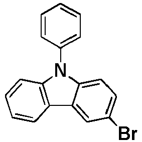 | 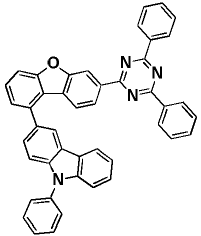 | 26% |
| 1-81 | | 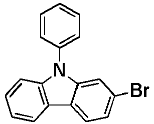 | 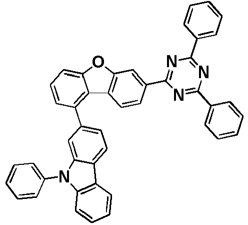 | 23% |
| 1-102 | | 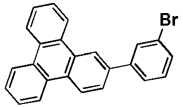 | 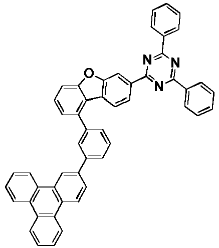 | 26% |
| 1-106 | | 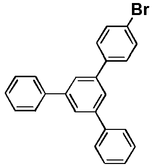 | 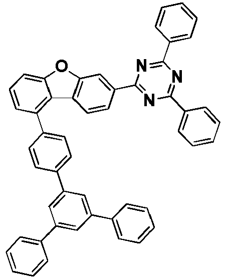 | 25% |

### <Preparation Example 2> Preparation of Compound 4-1 (not according to the invention)

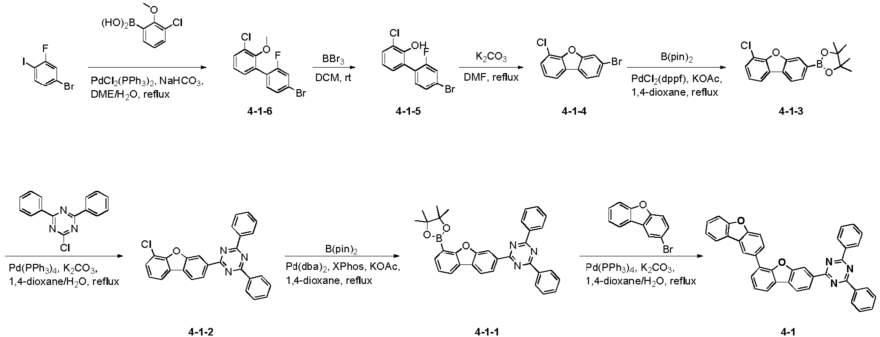

### 1) Preparation of Compound 4-1-6

After dissolving 4-bromo-2-fluoro-1-iodobenzene (200.0 g, 664.7 mM), (3-chloro-2-methoxyphenyl)boronic acid (148.7 g, 794.6 mM), Pd(PPh)₄ (38.4 g, 33.2 mM) and K₂CO₃ (183.7 g, 1329.4 mM) in 1,4-dioxane/H₂O (1 L/200 mL), the result was refluxed for 24 hours. After the reaction was completed, the result was extracted by introducing distilled water and DCM thereto at room temperature. The organic layer was dried with MgSO₄, and then the solvent was removed using a rotary evaporator. The reaction material was purified by column chromatography (DCM:Hex=1:4) to obtain target Compound 4-1-6 (169 g, 81%).

### 2) Preparation of Compound 4-1-5

After dissolving Compound 4-1-6 (169 g, 535.5 mM) and BBr₃ (103 mL, 1071.0 mM) in dichloromethane (DCM) (800 mL), the result was refluxed for 1 hour. After the reaction was completed, the result was extracted by introducing distilled water and DCM thereto at room temperature. The organic layer was dried with MgSO₄, and then the solvent was removed using a rotary evaporator. The reaction material was purified by column chromatography (DCM:Hex=1:1) to obtain target Compound 4-1-5 (145.3 g, 90%).

### 3) Preparation of Compound 4-1-4

After dissolving Compound 4-1-5 (145.3 g, 481.9 mM) and K₂CO₃ (133.2 g, 963.9 mM) in DMF (800 mL), the result was refluxed for 4 hours. After the reaction was completed, the result was extracted by introducing distilled water and DCM thereto at room temperature. The organic layer was dried with MgSO₄, and then the solvent was removed using a rotary evaporator. The reaction material was purified by column chromatography (DCM:Hex=1:8), and recrystallized with methanol to obtain target Compound 4-1-4 (74.6 g, 57%).

### 4) Preparation of Compound 4-1-3

After dissolving Compound 4-1-4 (74.6 g, 265.1 mM), bis(pinacolato)diboron (134.6 g, 530.2 mM), PdCl₂(dppf) (19.4 g, 26.5 mM) and KOAc (78.1 g, 795.3 mM) in 1,4-dioxane (500 mL), the result was refluxed for 24 hours. After the reaction was completed, the result was extracted by introducing distilled water and DCM thereto at room temperature. The organic layer was dried with MgSO₄, and then the solvent was removed using a rotary evaporator. The reaction material was purified by column chromatography (DCM:Hex=1:5) to obtain target Compound 4-1-3 (76.7 g, 88%).

### 5) Preparation of Compound 4-1-2

After dissolving Compound 4-1-3 (76.7 g, 233.28 mM), 2-chloro-4,6-diphenyl-1,3,5-triazine (67.7 g, 233.28 mM), Pd(PPh)₄ (13.5 g, 11.7 mM) and K₂CO₃ (64.5 g, 466.6 mM) in 1,4-dioxane/H₂O (800/160 mL), the result was refluxed for 24 hours. After the reaction was completed, the result was extracted by introducing distilled water and DCM thereto at room temperature. The organic layer was dried with MgSO₄, and then the solvent was removed using a rotary evaporator. The reaction material was purified by column chromatography (DCM:Hex=1:5), and recrystallized with methanol to obtain target Compound 4-1-2 (70.9 g, 70%).

### 6) Preparation of Compound 4-1-1

After dissolving Compound 4-1-2 (74.0 g, 163.3 mM), bis(pinacolato)diboron, 326.6 mM), Pd(dba)₂ (9.4 g, 16.3 mM), XPhos (15.6 g, 32.7 mM) and KOAc (48.1 g, 489.9 mM) in 1,4-dioxane (740 mL), the result was refluxed for 24 hours. After the reaction was completed, the result was extracted by introducing distilled water and DCM thereto at room temperature. The organic layer was dried with MgSO₄, and then the solvent was removed using a rotary evaporator. The reaction material was purified by column chromatography (DCM:Hex=1:4) to obtain target Compound 4-1-1 (72.9 g, 85%).

### 7) Preparation of Compound 4-1

After dissolving Compound 4-1-1 (15.0 g, 28.5 mM), 2-bromodibenzo[b,d]furan (7.8 g, 31.4 mM), Pd(PPh)₄ (1.6 g, 1.4 mM) and K₂CO₃ (7.9 g, 57.0 mM) in 1,4-dioxane/H₂O (200/40 mL), the result was refluxed for 24 hours. After the reaction was completed, the result was extracted by introducing distilled water and DCM thereto at room temperature. The organic layer was dried with MgSO₄, and then the solvent was removed using a rotary evaporator. The reaction material was purified by column chromatography (DCM:Hex=1:3), and recrystallized with methanol to obtain target Compound 4-1 (12.9 g, 80%).

Target Compound A was synthesized in the same manner as in Preparation Example 2 except that Intermediate A of the following Table 2 was used instead of 2-chloro-4,6-diphenyl-1,3,5-triazine, and Intermediate B of the following Table 2 was used instead of 2-bromodibenzo[b,d]furan.

**[Table 2](compounds 4-14, 4-37, 4-49, 4-61 and 4-81 not according to the invention)**

| Compou nd No. | Intermediate A | Intermediate B | Target Compound A | Yield |
|---|---|---|---|---|
| 4-2 | 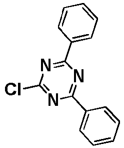 | 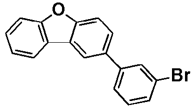 | 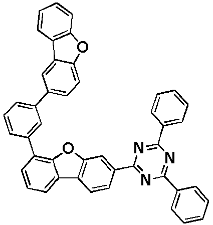 | 22% |
| 4-4 | | 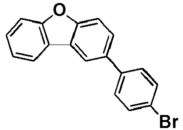 | 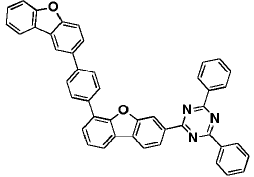 | 23% |
| 4-14 | | 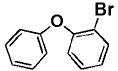 | 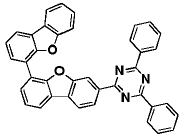 | 27% |
| 4-37 | | 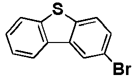 | 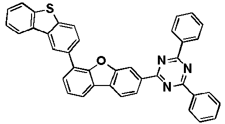 | 24% |
| 4-40 | | 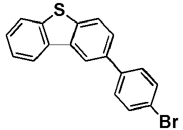 | 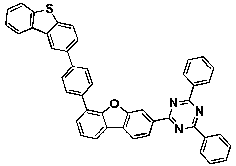 | 20% |
| 4-49 | | 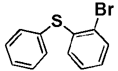 | 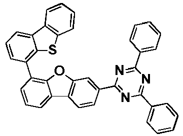 | 25% |
| 4-51 | | 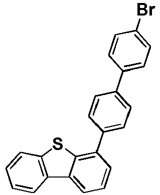 | 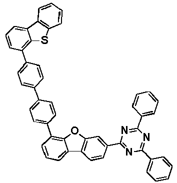 | 22% |
| 4-61 | | 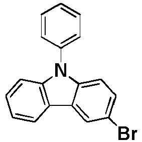 | 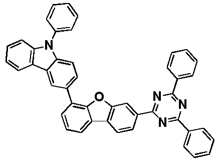 | 26% |
| 4-81 | | 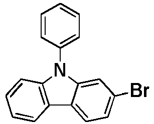 | 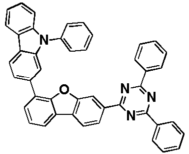 | 23% |

### <Preparation Example 3> Synthesis of Compound 5-3

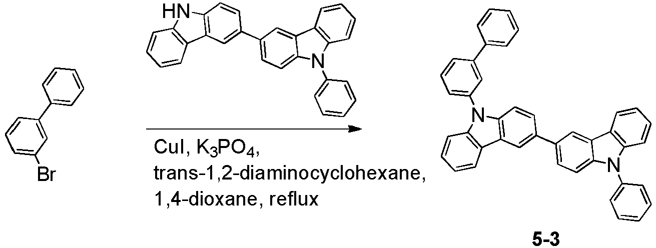

### 1) Preparation of Compound 5-3

After dissolving 3-bromo-1,1'-biphenyl (3.7 g, 15.8 mM), 9-phenyl-9H,9'H-3,3'-bicarbazole (6.5 g, 15.8 mM), CuI (3.0 g, 15.8 mM), trans-1,2-diaminocyclohexane (1.9 mL, 15.8 mM) and K₃PO₄ (3.3 g, 31.6 mM) in 1,4-dioxane (100 mL), the result was refluxed for 24 hours. After the reaction was completed, the result was extracted by introducing distilled water and DCM thereto at room temperature. The organic layer was dried with MgSO₄, and then the solvent was removed using a rotary evaporator. The reaction material was purified by column chromatography (DCM:Hex=1:3), and recrystallized with methanol to obtain target Compound 5-3 (7.5 g, 85%).

Target Compound A was synthesized in the same manner as in Preparation Example 3 except that Intermediate A of the following Table 3 was used instead of 3-bromo-1,1'-biphenyl, and Intermediate B of the following Table 3 was used instead of 9-phenyl-9H,9'H-3,3'-bicarbazole.

**[Table 3]**

| Compo und No. | Intermediate A | Intermediate B | Target Compound A | Total Yield |
|---|---|---|---|---|
| 5-4 | 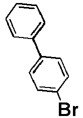 | 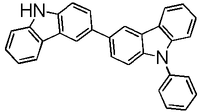 | 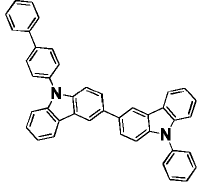 | 83% |
| 5-7 | 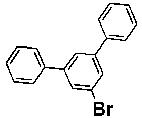 | | 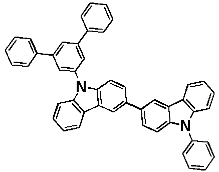 | 84% |
| 5-31 | 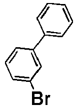 | 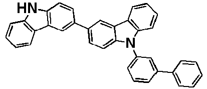 | 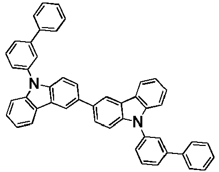 | 81% |
| 5-32 | 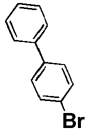 | | 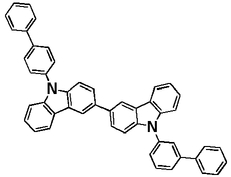 | 80% |
| 5-42 | 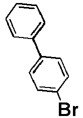 | 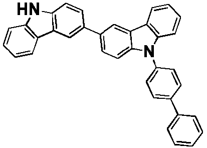 | 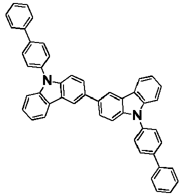 | 82% |

Heterocyclic compounds corresponding to the invention and Chemical Formula 2 other than the compounds described in Preparation Examples 1 to 3 and Tables 1 to 3 were also prepared in the same manner as in the methods described in the preparation examples described above.

Synthesis identification data of the compounds prepared above are as described in the following [Table 4] and [Table 5].

**[Table 4]**

| Compound No. | ¹H NMR (CDCl₃, 200 Mz) |
|---|---|
| 1-1 | δ=8.28(4H, d), 7.95(1H, d), 7.89(1H, d), 7.62-7.81(8H, m), 7.32-7.51(9H, m) |
| 1-2 | 5=8.28(4H, d), 7.95(1H, d), 7.89(1H, d), 7.32-7.81(21H, m) |
| 1-14 | δ=8.28(4H, d), 7.95(1H, d), 7.89(1H, d), 7.85(1H, d), 7.81(1H, d), 7.75(2H, d), 7.66(1H, d), 7.64(1H, s), 7.62 (1H, d), 7.32-7.51(10H, m) |
| 1-18 | δ=8.28(2H, d), 7.81-7.89(6H, m) 7.66(2H, d), 7.51-7.60(8H, m), 7.25-7.41(17H, m) |
| 1-37 | δ=8.45(1H, d), 8.28(4H, d), 7.95-8.00(4H, m), 7.86(1H, d), 7.75(2H, d), 7.64(1H, s), 7.62(1H, d), 7.41-7.52(9H, m) |
| 1-38 | δ=8.45(1H, d), 8.28(4H, d), 7.95-8.00(4H, m), 7.86(1H, d), 7.75(2H, d), 7.70(1H, s), 7.41-7.64(14H, m) |
| 1-49 | δ=8.45(1H, d), 8.41(1H, d), 8.28(4H, d), 8.20(1H, d), 7.98(1H, d), 7.95(1H, d), 7.75(2H, d), 7.41-7.64(12H, m) |
| 1-50 | δ=8.45(1H, d), 8.41(1H, d), 8.28(4H, d), 8.20(1H, d), 7.98(1H, d), 7.95(1H, d), 7.75(2H, d), 7.70(1H, s), 7.41-7.62(15H, m) |
| 1-61 | δ=8.28(4H, d), 8.18(1H, d), 8.12(1H, d), 8.00(1H, d), 7.95(1H, d), 7.77(1H, s), 7.75(2H, d), 7.41-7.77 (16H, m), 7.29(1H, t) |
| 1-81 | δ=8.49(1H, d), 8.28(4H, d), 8.12(1H, d), 8.10(1H, d), 7.95(1H, d), 7.75(2H, d), 7.41-7.64(17H, m), 7.29(1H, t) |
| 1-102 | δ=9.15(1H, s), 8.93(2H, d), 8.28(4H, d), 8.04-8.18(4H, m), 7.41-7.95(20H, m) |
| 1-106 | δ=8.28(4H, d), 7.95(1H, d), 7.75(2H, d), 7.62-7.66(5H, m), 7.41-7.52(17H, m), 7.25(4H, s) |
| 4-1 | δ =8.28(4H, d), 7.64-7.95(10H, m), 7.32-7.51(9H, m) |
| 4-2 | δ=8.36(4H, m), 8.08-7.73(11H, m), 7.61(2H, d), 7.54-7.50(8H, m), 7.39(1H, t), 7.31(1H, t) |
| 4-4 | δ =8.36(4H, m), 8.08-7.94(4H, m), 7.88(1H, d), 7.83-7.76(4H m), 7.54-7.50(8H, m), 7.39-7.25(6H, m), |
| 4-14 | δ=8.36(4H, m), 8.08(2H, d), 8.03-7.98(4H, m), 7.82(1H, d), 7.76(1H, s), 7.54-7.50(9H, m), 7.39(1H, t), 7.31(1H, t) |
| 4-37 | δ=8.45(1H, d), 8.36(4H, m), 8.12-7.99(6H, m), 7.93(1H, d), 7.82(1H, d), 7.76(1H, s), 7.56-7.49(9H, m), |
| 4-40 | δ=8.45(1H, d), 8.36(4H, m), 8.12-7.99(8H, m), 7.93(1H, d), 7.82(1H, d), 7.76(1H, d), 7.59-7.46(9H, m), 7.25(4H, s) |
| 4-49 | δ=8.55(1H, d), 8.45(1H, d), 8.36(4H, m), 8.08(1H, d), 8.03-8.02(2H, m), 7.93(1H, d), 7.82(1H, d) 7.76(1H, s), 7.70(1H, t), 7.56-7.49(10H, m) |
| 4-51 | δ=8.55(1H, d), 8.45(1H, d), 8.36-8.32(5H, m), 8.08(1H, d), 8.03-8.02(2H, m), 7.93(1H, d), 7.82(1H, d), 7.76(1H, s), 7.70(1H, t), 7.56-7.49(9H, m), 7.24(8H, s) |
| 4-61 | δ=8.36-8.30(5H, m), 8.19(1H, d), 8.13-8.03(4H, m), 7.89(1H, s), 7.82(1H, d), 7.76(1H, s), 7.62-7.48(14H, m), 7.20(1H, t) |
| 4-81 | δ=8.62(1H, d), 8.36(4H, m), 8.22(2H, t), 8.08-8.02(3H, m), 7.82(1H, d), 7.76(1H, s) 7.74(1H, s), 7.62-7.48(14H, m), 7.20(1H, t) |
| 5-3 | δ=8.55(1H, d), 8.30(1H, d), 8.21-8.13(3H, m), 7.99-7.89(3H, m), 7.77-7.35(17H, m), 7.20-7.16(2H, m) |
| 5-4 | δ=8.55(1H, d), 8.30(1H, d), 8.19-8.13(2H, m), 7.99-7.89(8H, m), 7.77-7.75(3H, m), 7.62-7.35(11H, m), 7.20-7.16(2H, m) |
| 5-7 | δ=8.55(1H, d), 8.31-8.30(3H, d), 8.19-8.13(2H, m), 7.99-7.89(5H, m), 7.77-7.75(5H, m), 7.62-7.35(14H, m), 7.20-7.16(2H, m) |
| 5-31 | δ=8.55(1H, d), 8.30(1H, d), 8.21-8.13(4H, m), 7.99-7.89(4H, m), 7.77-7.35(20H, m), 7.20-7.16(2H, m) |
| 5-32 | δ=8.55(1H, d), 8.30(1H, d), 8.21-8.13(3H, m), 7.99-7.89(8H, m), 7.77-7.35(17H, m), 7.20-7.16(2H, m) |
| 5-42 | δ=8.55(1H, d), 8.30(1H, d), 8.19(1H, d), 8.13(1H, d), 7.99-7.89(12H, m), 7.77-7.75(5H, m), 7.58(1H ,d), 7.49-7.35(8H, m), 7.20-7.16(2H, m) |

**[Table 5]**

| Compou nd | FD-MS | Compou nd | FD-MS |
|---|---|---|---|
| 1-1 | m/z=565.18(C₃₉H₂₃N₃O₂=56 5.62) | 1-2 | m/z=641.21(C₄₅H₂₇N₃O₂=64 1.71) |
| 1-14 | m/z=565.18(C₃₉H₂₃N₃O₂=56 5.62) | 1-18 | m/z=793.27(C₅₇H₃₅N₃O₂=79 3.91) |
| 1-37 | m/z=581.16(C₃₉H₂₃N₃OS=58 1.68) | 1-38 | m/z=657.19(C₄₅H₂₇N₃OS=65 7.78) |
| 1-49 | m/z=581.16(C₃₉H₂₃N₃OS=58 1.68) | 1-50 | m/z=657.19(C₄₅H₂₇N₃OS=65 7.78) |
| 1-61 | m/z=640.23(C₄₅H₂₈N₄O=640 .73) | 1-81 | m/z=640.23(C₄₅H₂₈N₄O=640 .73) |
| 1-102 | m/z=701.25(C₅₁H₃₁N₃O₀=70 1.81) | 1-106 | m/z=641.21(C₅₁H₃₃N₃O=641 .71) |
| 4-1 | m/z=565.18(C₃₉H₂₃N₃O₂=56 5.62) | 4-2 | m/z=641.21(C₄₅H₂₇N₃O₂=64 1.71) |
| 4-4 | m/z=641.21(C₄₅H₂₇N₃O₂=64 1.71) | 4-14 | m/z=565.18(C₃₉H₂₃N₃O₂=56 5.62) |
| 4-37 | m/z=581.16(C₃₉H₂₃N₃OS=58 1.68) | 4-40 | m/z=657.19(C₄₅H₂₇N₃OS=65 7.78) |
| 4-49 | m/z=581.16(C₃₉H₂₃N₃OS=58 1.68) | 4-51 | m/z=733.22(C₅₁H₃₁N₃OS=73 3.88) |
| 4-61 | m/z=640.23(C₄₅H₂₈N₄O=640 .73) | 4-81 | m/z=640.23(C₄₅H₂₈N₄O=640 .73) |
| 5-3 | m/z=560.23 (C₄₂H₂₈N₂=560.70) | 5-4 | m/z=560.23 (C₄₂H₂₈N₂=560.70) |
| 5-7 | m/z=636.26 (C₄₈H₃₂N₂=636.80) | 5-31 | m/z=636.26 (C₄₈H₃₂N₂=636.80) |
| 5-32 | m/z=636.26 (C₄₈H₃₂N₂=636.80) | 5-42 | m/z=636.26 (C₄₈H₃₂N₂=636.78) |

### <Experimental Example 1>-Manufacture of Organic Light Emitting Device

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and ultraviolet ozone (UVO) treatment was conducted for 5 minutes using UV in a UV cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl (phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. As the light emitting layer, a compound of the following Table 6 was deposited to 400 Å as a host, and as a green phosphorescent dopant, Ir(ppy)₃ was doped and deposited by 7% with respect to the deposited thickness of the light emitting layer. After that, BCP was deposited to 60 Å as a hole blocking layer, and Alq₃ was deposited to 200 Å thereon as an electron transfer layer. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic electroluminescent device was manufactured.

Meanwhile, all the organic compounds required to manufacture the OLED were vacuum sublimation purified under 1.3 × 10⁻⁶ pascals to 1.3 × 10⁻⁴ pascals (10⁻⁸ torr to 10⁻⁶ torr) for each material to be used in the OLED manufacture.

For each of the organic electroluminescent devices manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc..

Results of measuring driving voltage, light emission efficiency, color coordinate (CIE) and lifetime of the manufactured organic light emitting devices are as shown in the following Table 6.

**[Table 6]**

| | Light Emitting Layer Compound | Driving Voltage (V) | Efficiency (cd/A) | Color Coordinate (x, y) | Lifetime (T₉₀) |
|---|---|---|---|---|---|
| Comparative Example 1 | 5-3 | 2.63 | 29.2 | (0.233, 0.671) | 45 |
| Comparative Example 2 | 5-4 | 2.59 | 31.3 | (0.231, 0.673) | 48 |
| Comparative Example 3 | 5-7 | 2.61 | 30.4 | (0.237, 0.677) | 44 |
| Comparative Example 4 | 5-31 | 2.65 | 30.8 | (0.234, 0.674) | 43 |
| Comparative Example 5 | 5-32 | 2.58 | 29.8 | (0.231, 0.681) | 44 |
| Comparative Example 6 | 5-42 | 2.59 | 30.1 | (0.233, 0.682) | 43 |
| Comparative Example 7 | Ref. 1 | 6.14 | 38.9 | (0.236, 0.687) | 133 |
| Comparative Example 8 | Ref. 2 | 6.54 | 35.3 | (0.236, 0.666) | 69 |
| Comparative Example 9 | Ref. 3 | 4.85 | 43.4 | (0.237, 0.677) | 85 |
| Comparative Example 10 | Ref. 4 | 2.91 | 29.7 | (0.233, 0.683) | 50 |
| Comparative Example 11 | Ref. 5 | 6.66 | 35.8 | (0.233, 0.681) | 64 |
| Comparative Example 12 | Ref. 6 | 6.15 | 34.9 | (0.231, 0.671) | 77 |
| Comparative Example 13 | Ref. 7 | 6.30 | 30.5 | (0.233, 0.678) | 64 |
| Comparative Example 14 | Ref. 8 | 5.54 | 43.9 | (0.241, 0.682) | 175 |
| Comparative Example 15 | Ref. 9 | 5.84 | 49.4 | (0.241, 0.673) | 185 |
| Comparative Example 16 | Ref. 10 | 5.31 | 45.9 | (0.240, 0.681) | 173 |
| Example 1 | 1-1 | 4.31 | 53.2 | (0.247, 0.667) | 227 |
| Example 2 | 1-2 | 4.30 | 55.8 | (0.241, 0.671) | 224 |
| Example 3 | 1-14 | 4.45 | 52.7 | (0.251, 0.674) | 225 |
| Example 4 | 1-18 | 4.38 | 54.0 | (0.240, 0.672) | 228 |
| Example 5 | 1-37 | 4.34 | 54.1 | (0.242, 0.673) | 232 |
| Example 6 | 1-38 | 4.31 | 55.2 | (0.231, 0.681) | 238 |
| Example 7 | 1-49 | 4.41 | 53.7 | (0.241, 0.683) | 233 |
| Example 8 | 1-50 | 4.39 | 55.0 | (0.231, 0.674) | 241 |
| Example 9 | 1-61 | 4.15 | 50.4 | (0.231, 0.684) | 195 |
| Example 10 | 1-81 | 4.12 | 50.8 | (0.246, 0.677) | 190 |
| Example 11 | 1-102 | 4.42 | 55.7 | (0.239, 0.682) | 222 |
| Example 12 | 1-106 | 4.27 | 54.1 | (0.243, 0.671) | 220 |
| Example 13 | 4-1 | 4.20 | 55.8 | (0.247, 0.685) | 259 |
| Example 14 | 4-2 | 4.21 | 57.9 | (0.241, 0.668) | 261 |
| Example 15 | 4-4 | 4.29 | 55.3 | (0.251, 0.672) | 262 |
| Example 16 | 4-14 | 4.28 | 56.0 | (0.240, 0.674) | 260 |
| Example 17 | 4-37 | 4.19 | 57.3 | (0.242, 0.683) | 264 |
| Example 18 | 4-40 | 4.21 | 56.5 | (0.231, 0.667) | 270 |
| Example 19 | 4-49 | 4.31 | 55.6 | (0.241, 0.671) | 261 |
| Example 20 | 4-51 | 4.27 | 55.7 | (0.231, 0.682) | 273 |
| Example 21 | 4-61 | 3.99 | 51.1 | (0.231, 0.677) | 211 |
| Example 22 | 4-81 | 4.01 | 50.3 | (0.246, 0.673) | 206 |

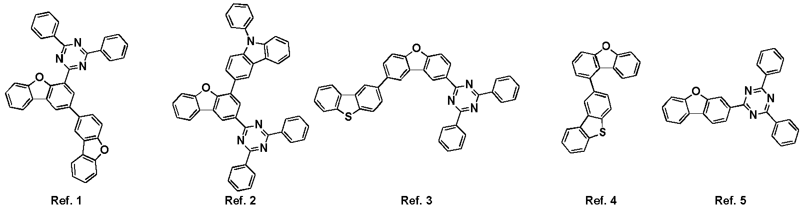

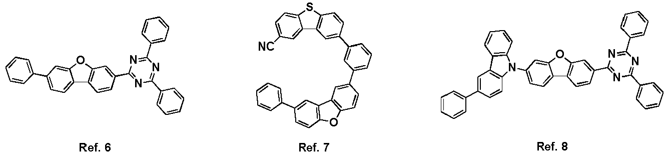

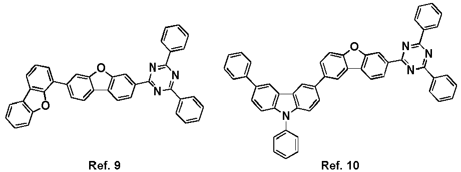

### <Experimental Example 2>-Manufacture of Organic Light Emitting Device

A glass substrate on which ITO was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO treatment was conducted for 5 minutes using UV in a UV cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl (phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. As the light emitting layer, one type of compound of the invention and one type of compound described as Chemical Formula 2 as in the following Table 7 were premixed and then deposited in one source of supply to 400 Å as a host, and as a green phosphorescent dopant, Ir(ppy)₃ was doped and deposited by 7% with respect to the deposited thickness of the light emitting layer. After that, BCP was deposited to 60 Å as a hole blocking layer, and Alq₃ was deposited to 200 Å thereon as an electron transfer layer. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic electroluminescent device was manufactured.

Meanwhile, all the organic compounds required to manufacture the OLED were vacuum sublimation purified under 1.3 × 10⁻⁶ pascals to 1.3 × 10⁻⁴ pascals (10⁻⁸ torr to 10⁻⁶ torr) for each material to be used in the OLED manufacture.

For each of the organic electroluminescent devices manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc..

Results of measuring driving voltage, light emission efficiency, color coordinate (CIE) and lifetime of the organic light emitting devices manufactured according to the present disclosure are as shown in the following Table 7.

**[Table 7]**

| | Light Emitting Layer Compound | Ratio | Driving Voltage (V) | Efficiency (cd/A) | Color Coordinate (x, y) | Lifetime (T₉₀) |
|---|---|---|---|---|---|---|
| Example 43 | 1-1:5-3 | 1:8 | 4.77 | 55.2 | (0.233, 0.714) | 314 |
| Example 44 | | 1:5 | 4.55 | 57.2 | (0.243, 0.714) | 355 |
| Example 45 | | 1:2 | 4.13 | 75.3 | (0.241, 0.714) | 497 |
| Example 46 | | 1:1 | 3.81 | 74.9 | (0.231, 0.711) | 481 |
| Example 47 | | 2:1 | 3.88 | 71.2 | (0.251, 0.714) | 470 |
| Example 48 | | 5:1 | 4.33 | 68.3 | (0.241, 0.711) | 401 |
| Example 49 | | 8:1 | 4.64 | 51.0 | (0.247, 0.727) | 361 |
| Example 50 | 1-1:5-4 | 1:3 | 4.51 | 66.2 | (0.243, 0.714) | 405 |
| Example 51 | | 1:2 | 4.20 | 76.3 | (0.241, 0.714) | 490 |
| Example 52 | | 1:1 | 3.91 | 75.1 | (0.233, 0.712) | 481 |
| Example 53 | | 2:1 | 3.96 | 73.4 | (0.251, 0.712) | 462 |
| Example 54 | | 3:1 | 4.23 | 70.9 | (0.247, 0.711) | 434 |
| Example 55 | 1-49:5-7 | 1:2 | 4.18 | 77.2 | (0.250, 0.714) | 505 |
| Example 56 | | 1:1 | 3.82 | 78.4 | (0.231, 0.714) | 527 |
| Example 57 | | 2:1 | 3.90 | 76.8 | (0.251, 0.712) | 472 |
| Example 58 | 1-102:5-31 | 1:2 | 4.02 | 73.7 | (0.242, 0.722) | 492 |
| Example 59 | | 1:1 | 3.89 | 75.2 | (0.231, 0.711) | 513 |
| Example 60 | | 2:1 | 4.08 | 74.1 | (0.251, 0.724) | 485 |
| Example 61 | 4-1:5-31 | 1:2 | 4.11 | 79.1 | (0.242, 0.724) | 566 |
| Example 62 | | 1:1 | 3.95 | 89.5 | (0.231, 0.711) | 624 |
| Example 63 | | 2:1 | 3.86 | 77.3 | (0.251, 0.711) | 540 |
| Example 64 | 4-1:5-42 | 1:2 | 4.12 | 79.4 | (0.241, 0.712) | 575 |
| Example 65 | | 1:1 | 3.69 | 87.7 | (0.251, 0.714) | 618 |
| Example 66 | | 2:1 | 3.79 | 76.3 | (0.233, 0.714) | 542 |
| Example 67 | 4-14:5-32 | 1:2 | 4.10 | 78.1 | (0.231, 0.712) | 581 |
| Example 68 | | 1:1 | 3.85 | 85.9 | (0.231, 0.711) | 610 |
| Example 69 | | 2:1 | 3.86 | 76.2 | (0.251, 0.712) | 557 |
| Example 70 | 4-14:5-42 | 1:2 | 4.08 | 77.4 | (0.247, 0.710) | 562 |
| Example 71 | | 1:1 | 3.81 | 81.0 | (0.243, 0.712) | 603 |
| Example 72 | | 2:1 | 3.80 | 78.4 | (0.232, 0.714) | 542 |
| Example 73 | 4-49:5-32 | 1:2 | 3.99 | 78.1 | (0.241, 0.714) | 547 |
| Example 74 | | 1:1 | 3.75 | 80.2 | (0.251, 0.720) | 610 |
| Example 75 | | 2:1 | 3.77 | 76.1 | (0.251, 0.715) | 516 |
| Example 76 | 4-61:5-42 | 1:2 | 4.87 | 76.7 | (0.232, 0.714) | 551 |
| Example 77 | | 1:1 | 4.01 | 77.9 | (0.231, 0.714) | 575 |
| Example 78 | | 2:1 | 4.23 | 75.2 | (0.235, 0.711) | 547 |
| Comparative Example 17 | Ref. 8: 5-32 | 1:2 | 3.87 | 72.7 | (0.231, 0.714) | 440 |
| Comparative Example 18 | | 1:1 | 3.95 | 71.5 | (0.242, 0.711) | 480 |
| Comparative Example 19 | | 2:1 | 3.61 | 77.9 | (0.231, 0.712) | 498 |

As seen from the results of Table 6, it was identified that the organic electroluminescent device using the light emitting layer material of the organic electroluminescent device of the present disclosure had lower driving voltage, enhanced light emission efficiency and significantly improved lifetime compared to Comparative Examples 7 to 14.

It was identified that, in Examples 11 and 12 having an aryl group substituting another benzene ring not substituted with the N-containing ring in the dibenzofuran structure, a T1 energy level higher by approximately 2.5 eV or greater was obtained, which readily transferred energy from the host to the dopant, and superior light emission efficiency was obtained as in the arylene group or heteroarylene group-substituted chemical formulae.

Particularly, it was identified that, although the HOMO energy level was localized to one side when X1 has a substituent of NR13, the HOMO energy level was relatively delocalized when X1 has O, S and the like, which leads to a more stable electron-stable structure, and an organic light emitting device with improved lifetime, driving stability and efficiency was manufactured.

It was identified that, when the No. 3 position of one side benzene ring of the dibenzofuran is substituted with N-Het and the No. 4 position of another benzene ring has a specific substituent, a driving voltage was low due to particularly more favorable current density compared to cases of substituting other positions, and triplet energy was also high. It was identified that, when the No. 3 position of one side benzene ring of the dibenzofuran is substituted with N-Het and the No. 1 position of another benzene ring has a specific substituent, thermal stability was superior due to particularly lower Td compared to cases of substituting other positions, and lifetime properties of the organic light emitting device were particularly superior.

In addition, as seen from the results of Tables 6 and 7, effects of more superior efficiency and lifetime were obtained when comprising the compound of the invention and the compound of Chemical Formula 2 at the same time. Such results may lead to a forecast that an exciplex phenomenon occurred when comprising the two compounds at the same time.

The exciplex phenomenon is a phenomenon of releasing energy having sizes of a donor (p-host) HOMO level and an acceptor (n-host) LUMO level due to electron exchanges between two molecules. When the exciplex phenomenon occurs between two molecules, reverse intersystem crossing (RISC) occurs, and as a result, internal quantum efficiency of fluorescence may increase up to 100%. When a donor (p-host) having a favorable hole transfer ability and an acceptor (n-host) having a favorable electron transfer ability are used as a host of a light emitting layer, holes are injected to the p-host and electrons are injected to the n-host, and therefore, a driving voltage may be lowered, which resultantly helps with enhancement in the lifetime. In the invention of the present application, it was identified that excellent device properties were obtained when, as the light emitting layer host, the compound of Chemical Formula 2 performing a donor role and the compound of the invention performing an acceptor role were used.

The compounds of Comparative Examples 7, 8 and 9 had a different position of substitution from the compound of the present disclosure, and in the compounds of Comparative Examples 10 and 11, one of the two substituents having the dibenzofuran structure of the invention was not present, and it was identified that this broke a balance between holes and electrons in the light emitting layer leading to a decrease in the lifetime. In addition, it was identified that, when the N portion of carbazole bonds to the dibenzofuran as in the compound of Comparative Example 14, holes moved faster leading to a decrease in the lifetime.

## Claims

1. A heterocyclic compound represented by any one of the following compounds:

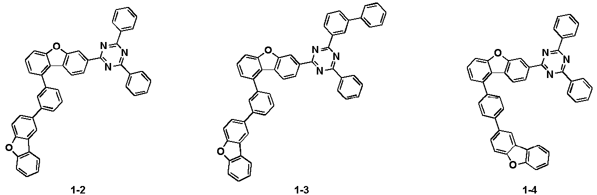

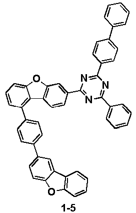

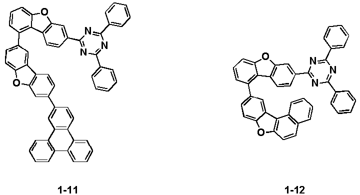

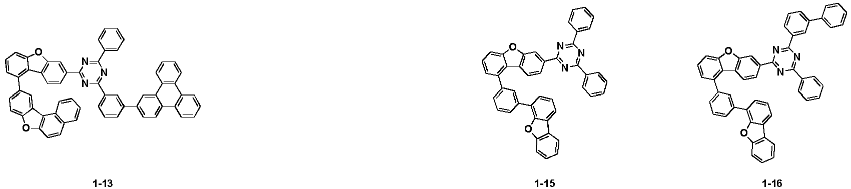

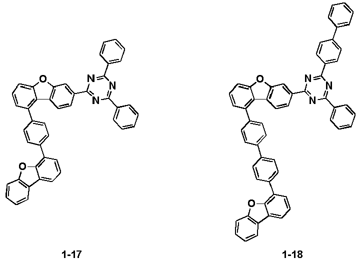

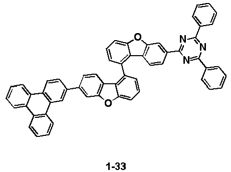

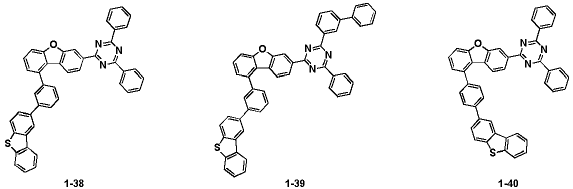

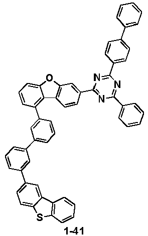

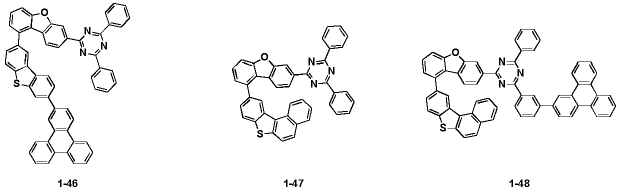

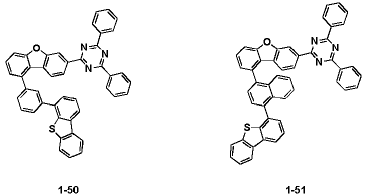

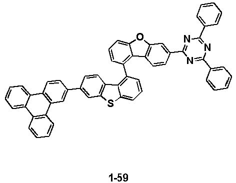

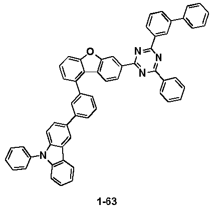

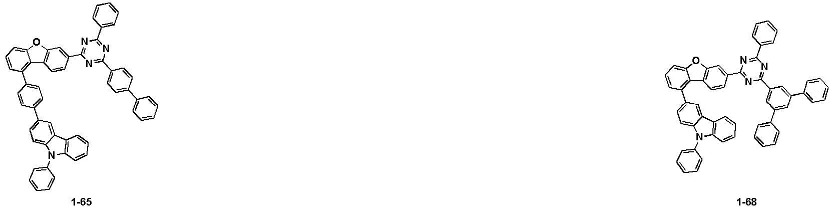

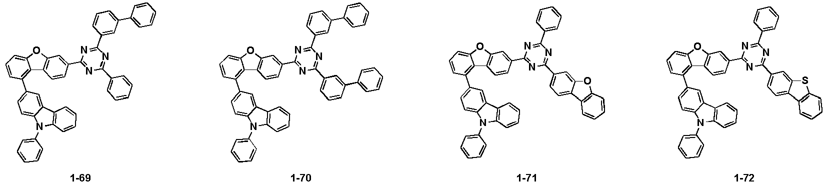

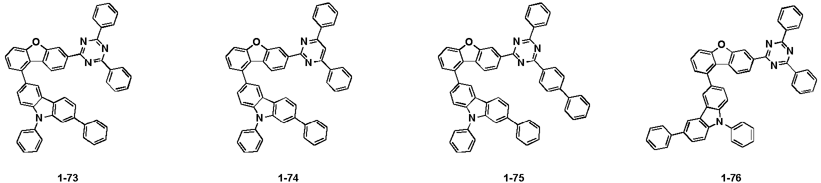

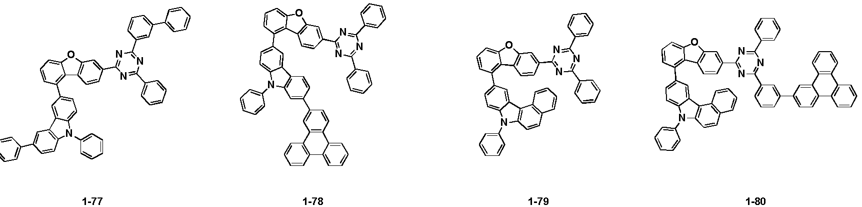

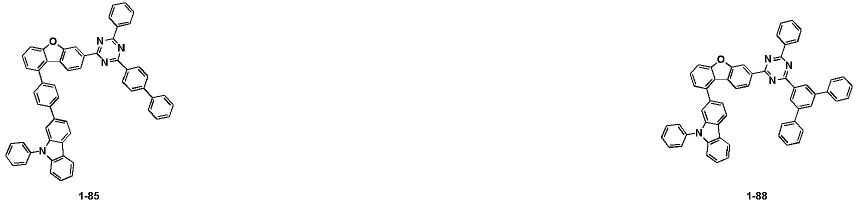

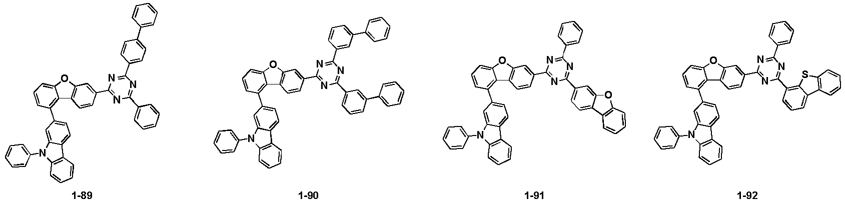

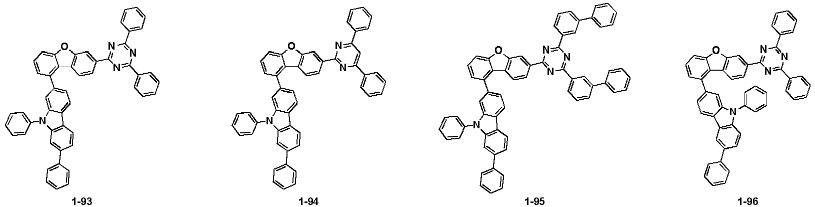

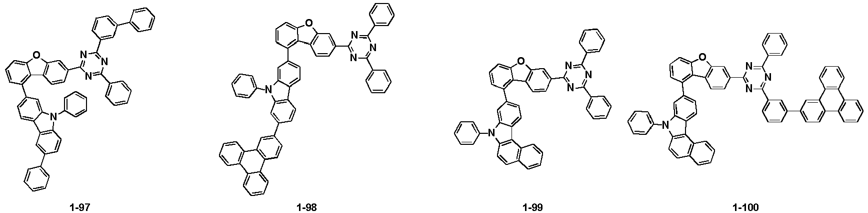

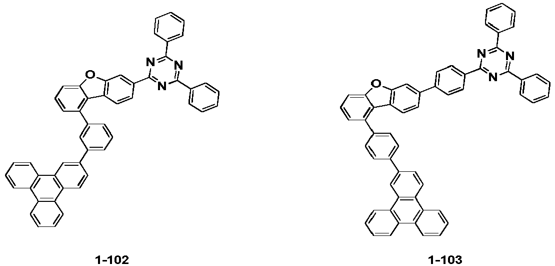

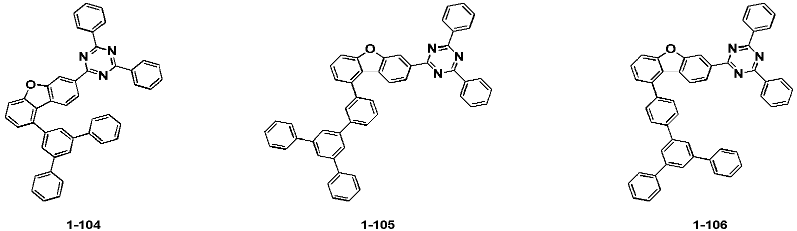

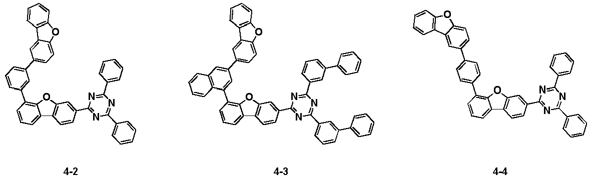

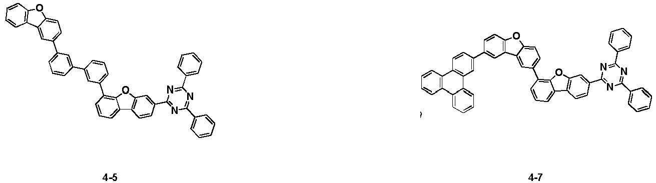

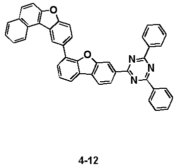

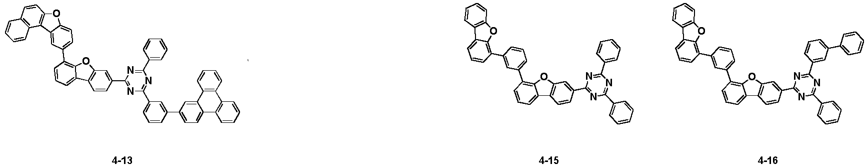

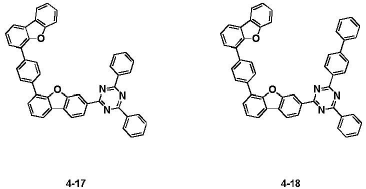

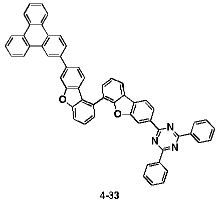

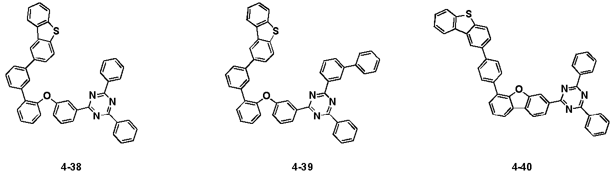

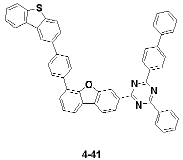

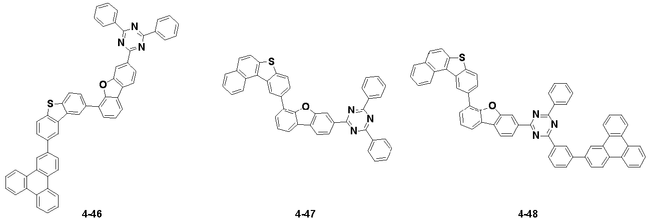

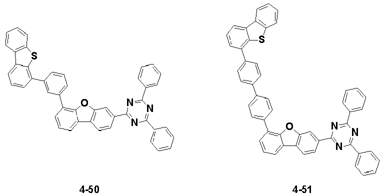

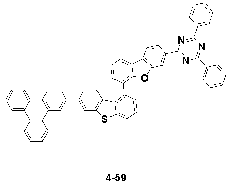

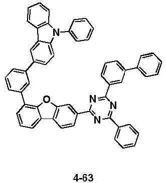

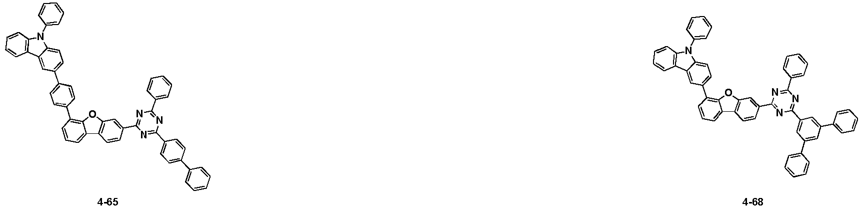

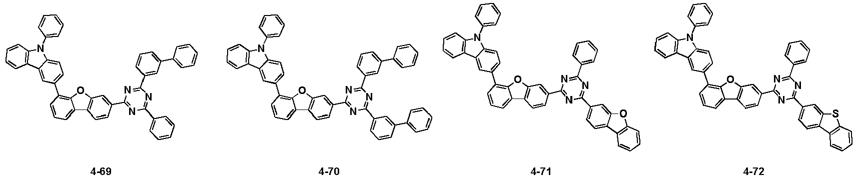

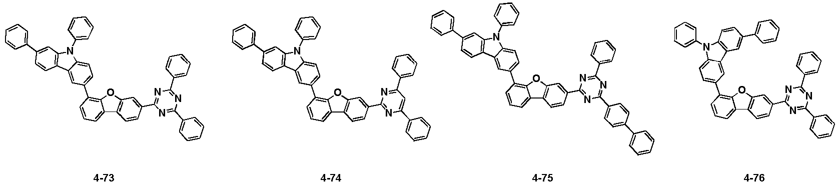

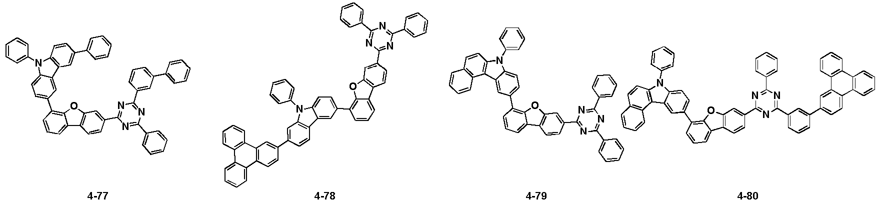

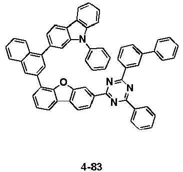

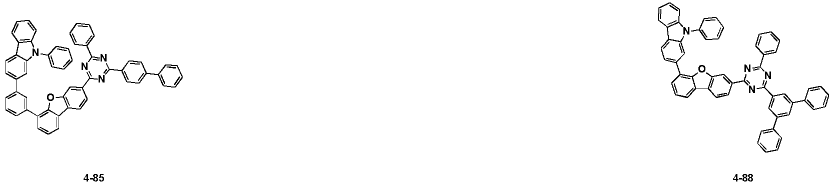

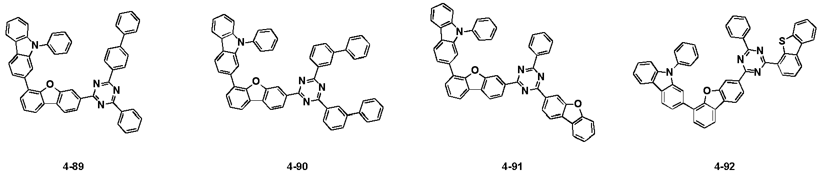

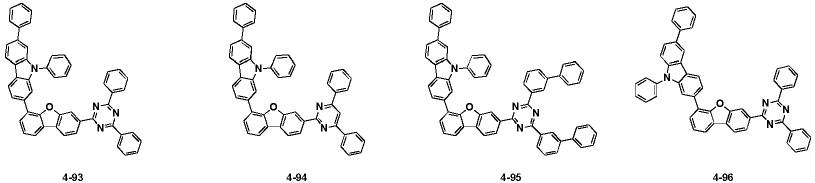

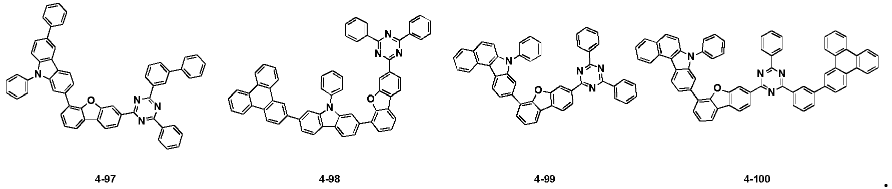

2. An organic light emitting device comprising:
a first electrode;
a second electrode provided opposite to the first electrode; and
one or more organic material layers provided between the first electrode and the second electrode,
wherein one or more layers of the organic material layers comprise the heterocyclic compound of claim 1.

3. The organic light emitting device of Claim 2, wherein the organic material layer comprises a light emitting layer, an electron injection layer, an electron transfer layer, an electron blocking layer or a hole blocking layer and the light emitting layer, an electron injection layer, an electron transfer layer, an electron blocking layer or a hole blocking layer comprises the heterocyclic compound.

4. The organic light emitting device Claim 2, further comprising one, two or more layers selected from the group consisting of a light emitting layer, a hole injection layer, a hole transfer layer. an electron injection layer, an electron transfer layer, an electron blocking layer and a hole blocking layer.

5. The organic light emitting device of Claim 2, wherein the organic material layer further comprises a compound of the following Chemical Formula 2: 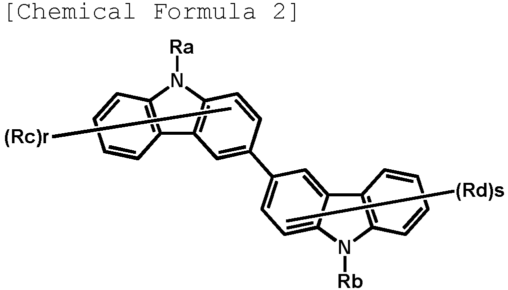 in Chemical Formula 2,
Ra and Rb are the same as or different from each other, and each independently a substituted or unsubstituted C6 to C60 aryl group; or a substituted or unsubstituted C2 to C60 heteroaryl group;
Rc and Rd are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; and a substituted or unsubstituted amine group; and
r and s are an integer of 0 to 7.

6. The organic light emitting device of Claim 5, wherein Chemical Formula 2 is represented by any one of the following compounds:

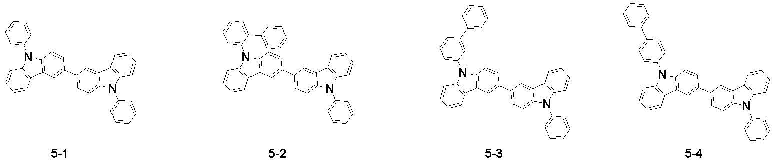

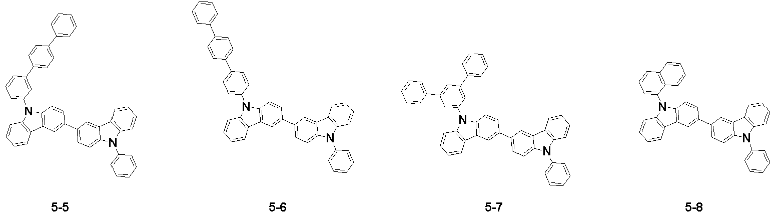

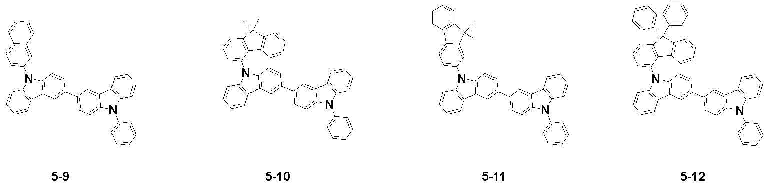

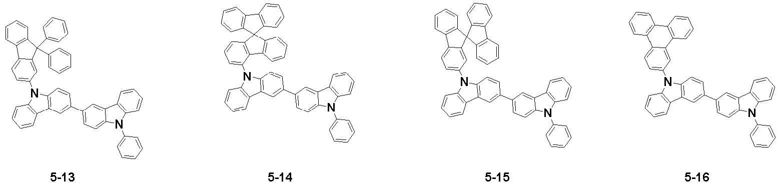

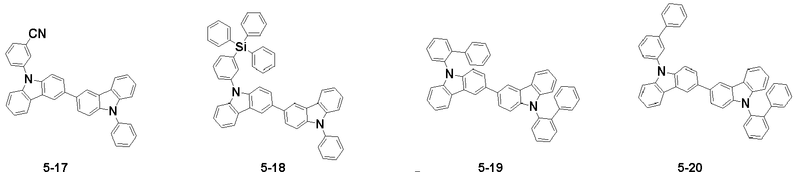

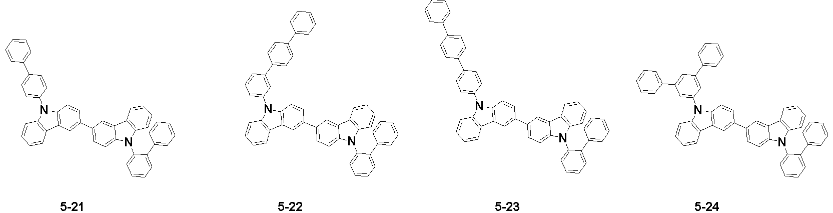

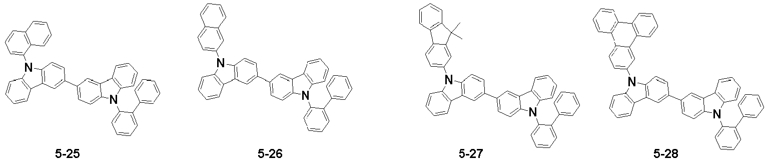

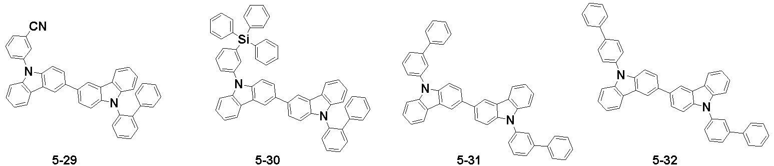

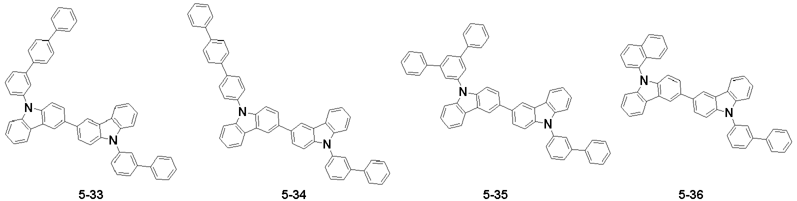

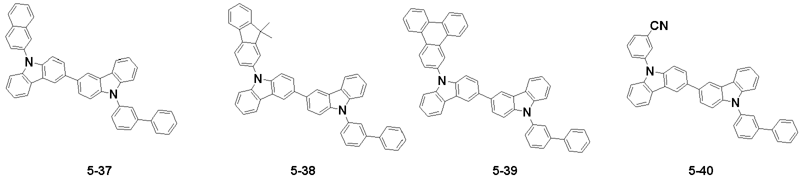

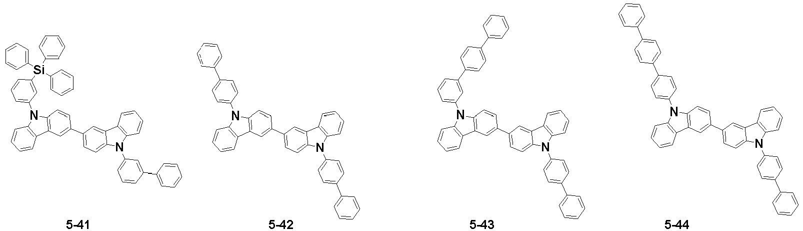

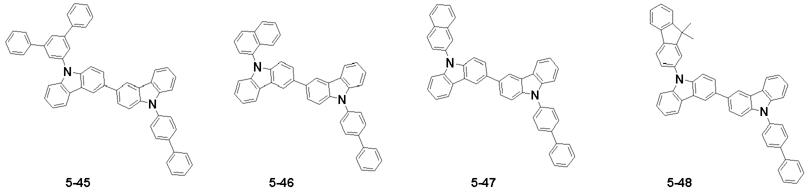

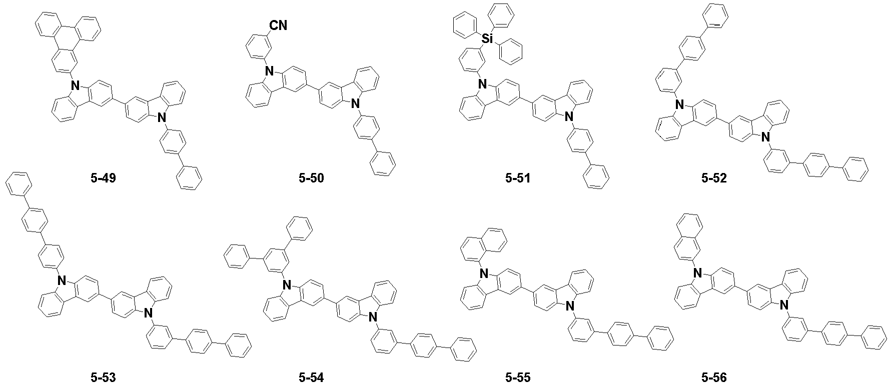

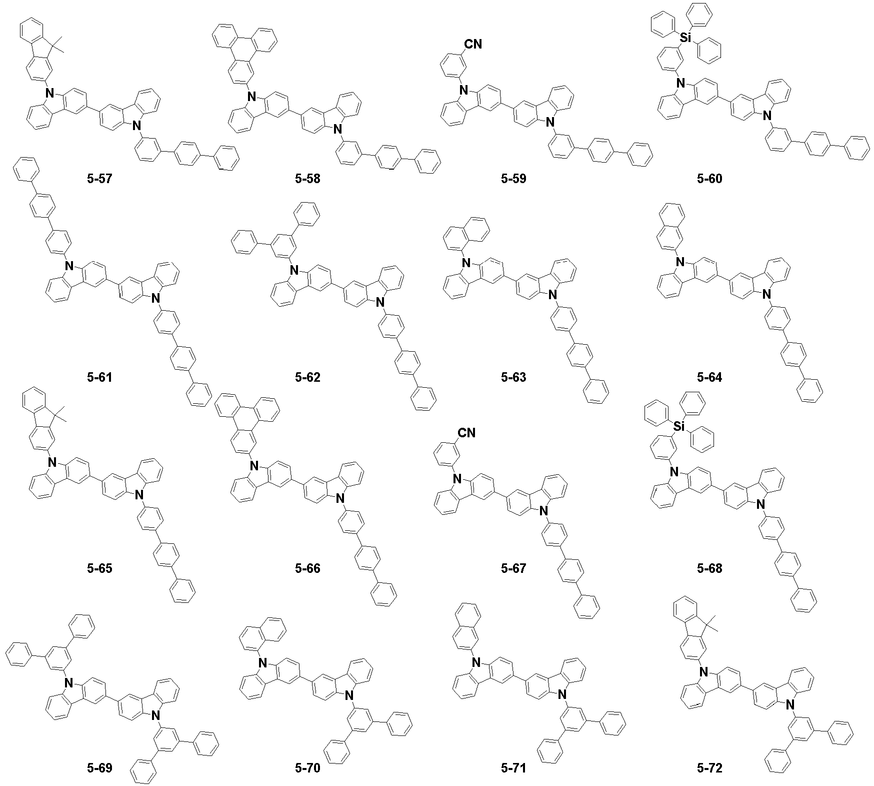

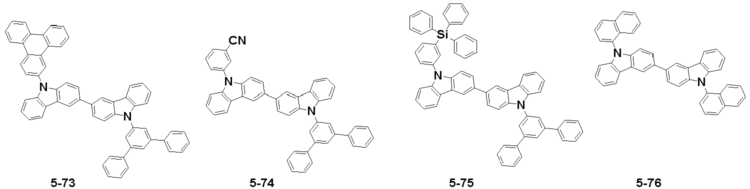

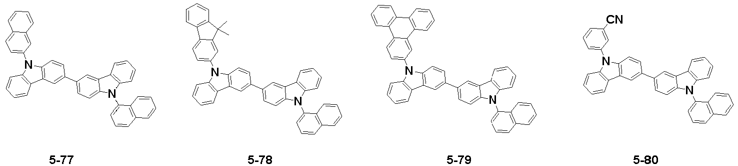

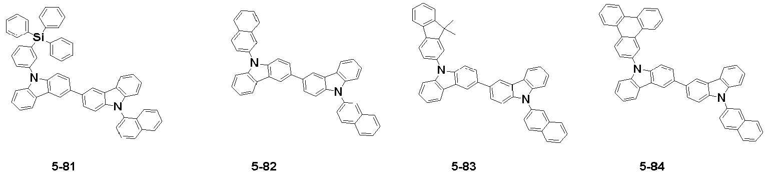

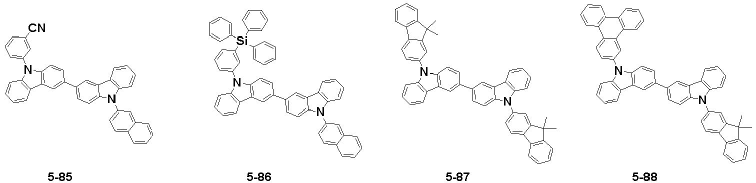

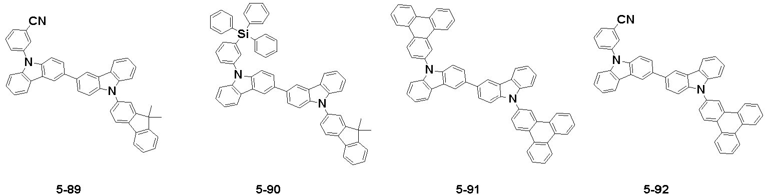

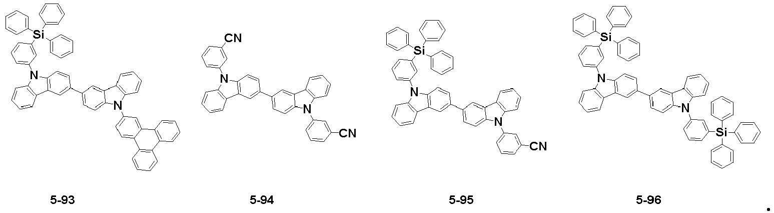

## Patentansprüche

1. Heterocyclische Verbindung, dargestellt durch eine der folgenden Verbindungen:

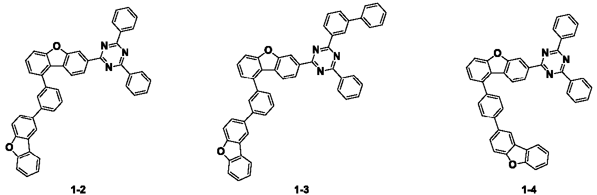

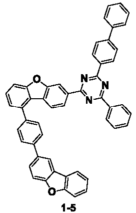

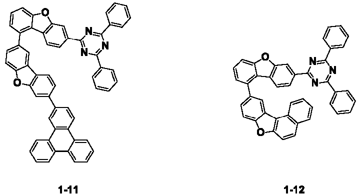

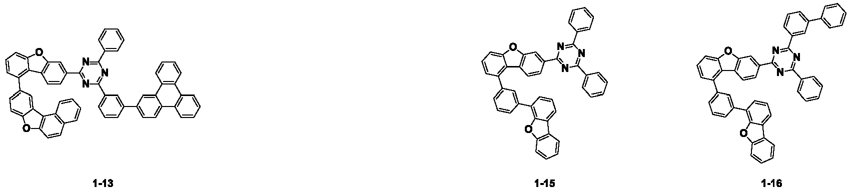

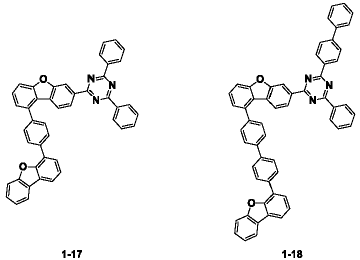

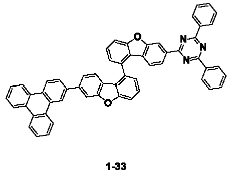

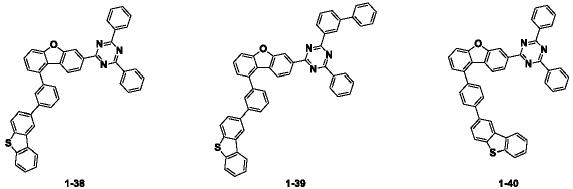

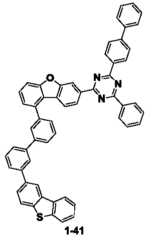

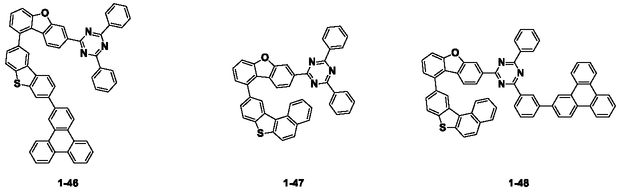

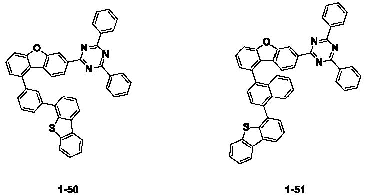

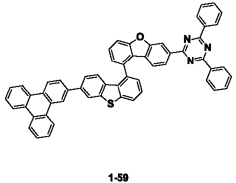

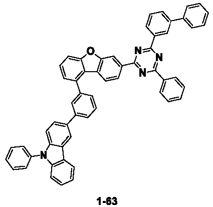

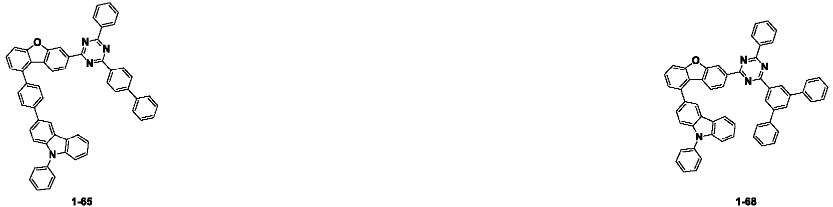

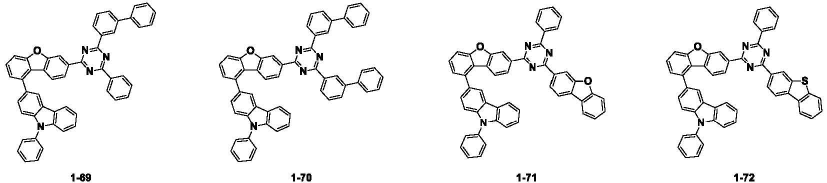

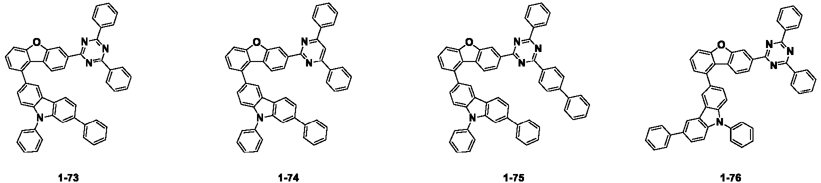

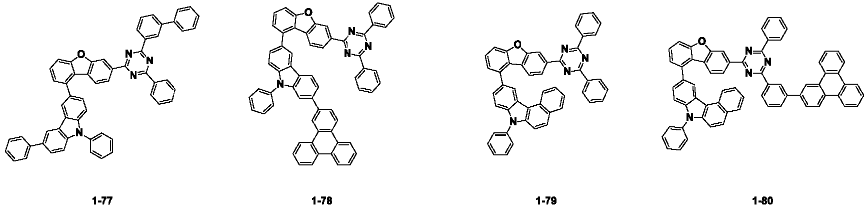

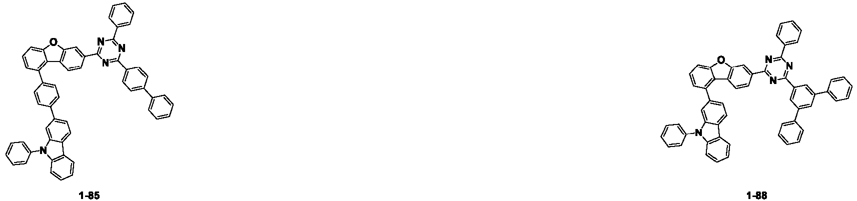

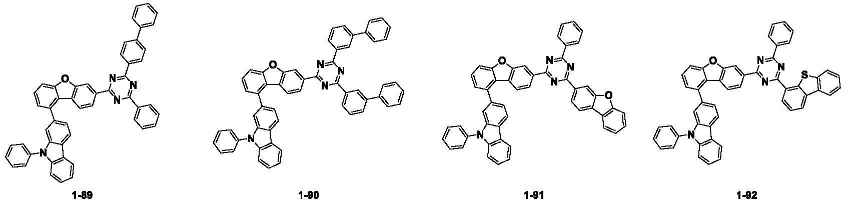

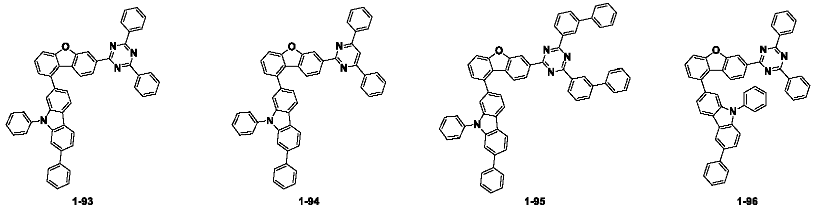

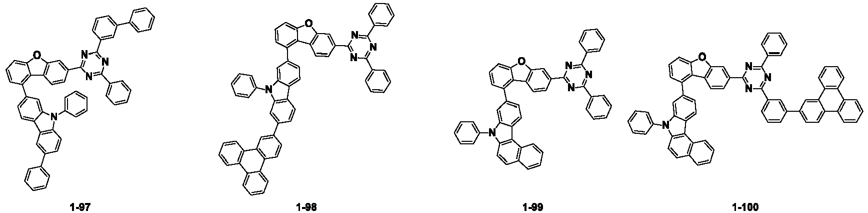

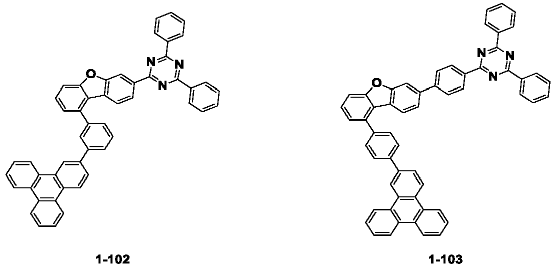

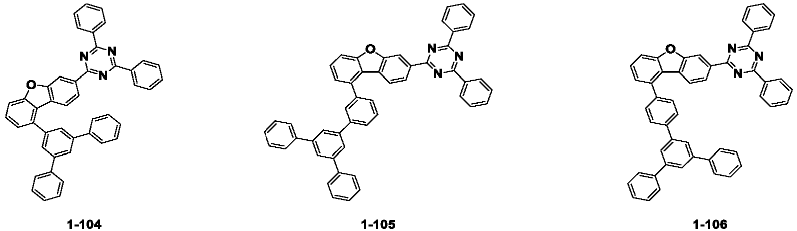

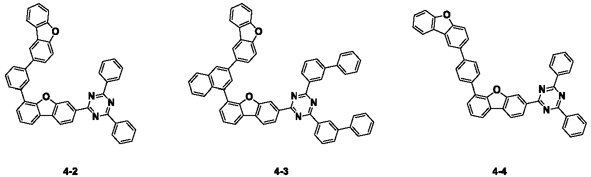

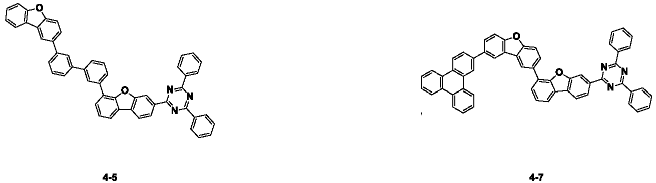

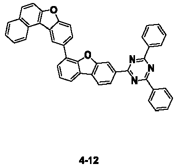

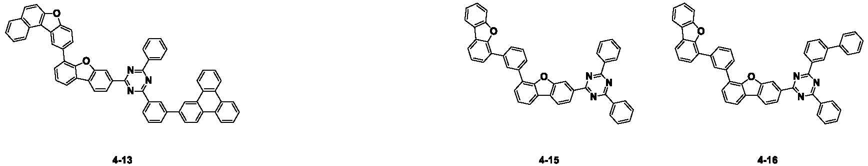

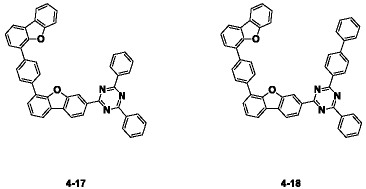

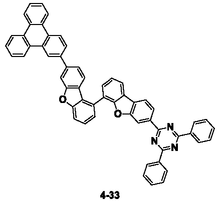

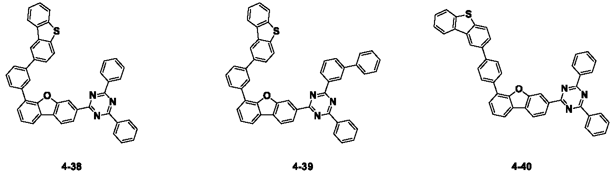

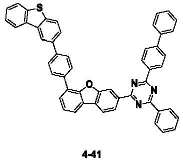

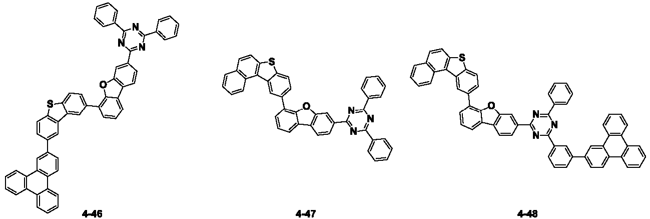

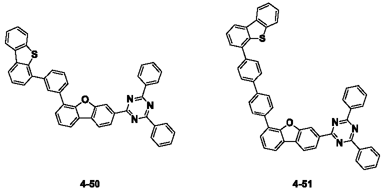

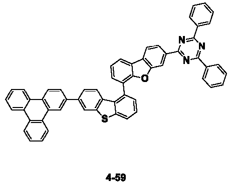

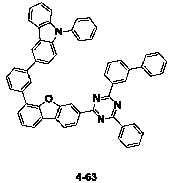

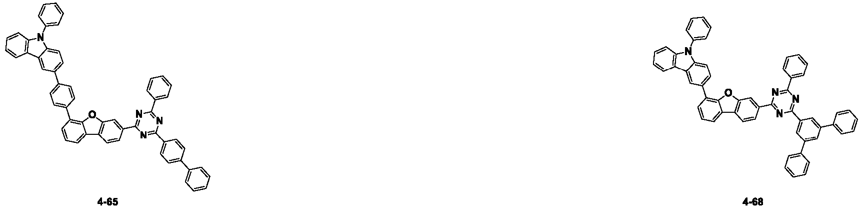

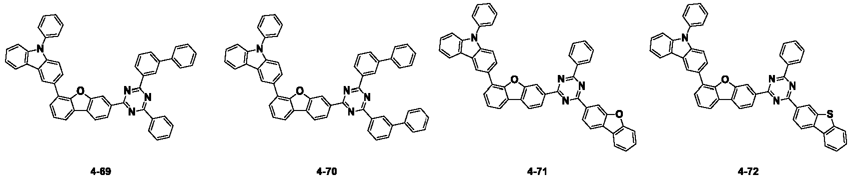

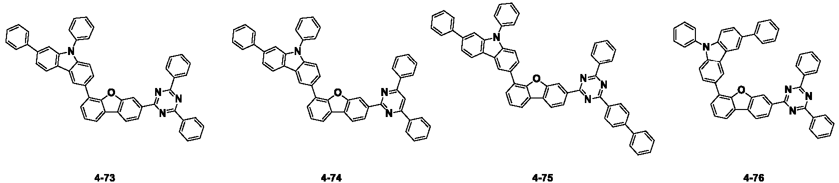

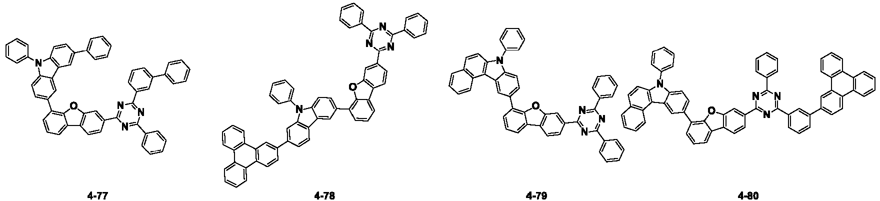

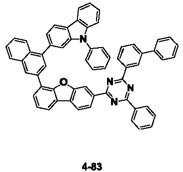

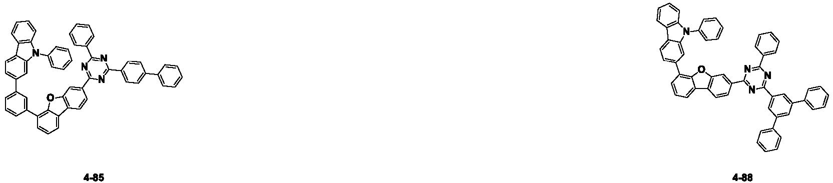

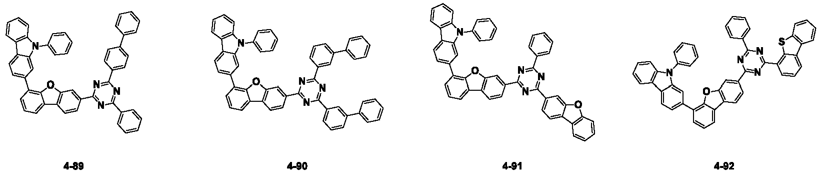

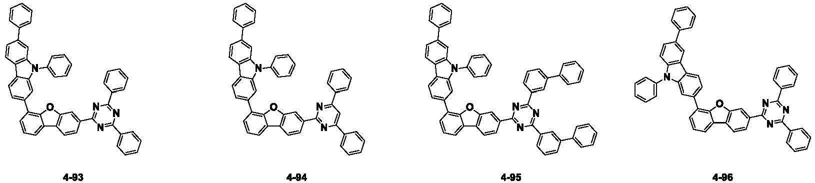

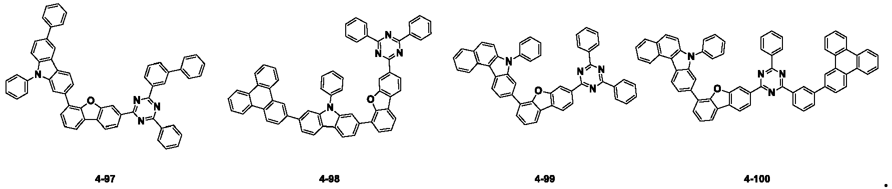

2. Organische lichtemittierende Vorrichtung, umfassend:
eine erste Elektrode;
eine zweite Elektrode, die gegenüber der ersten Elektrode bereitgestellt ist; und
eine oder mehrere organische Materialschichten, die zwischen der ersten Elektrode und der zweiten Elektrode bereitgestellt sind,
wobei eine oder mehrere Schichten der organischen Materialschichten die heterocyclische Verbindung nach Anspruch 1 umfassen.

3. Organische lichtemittierende Vorrichtung nach Anspruch 2, wobei die organische Materialschicht eine lichtemittierende Schicht, eine Elektroneninjektionsschicht, eine Elektronentransferschicht, eine Elektronenblockierschicht oder eine Lochblockierschicht umfasst und die lichtemittierende Schicht, eine Elektroneninjektionsschicht, eine Elektronentransferschicht, eine Elektronenblockierschicht oder eine Lochblockierschicht die heterocyclische Verbindung umfasst.

4. Organische lichtemittierende Vorrichtung nach Anspruch 2, ferner umfassend eine, zwei oder mehr Schichten, ausgewählt aus der Gruppe bestehend aus einer lichtemittierenden Schicht, einer Lochinjektionsschicht, einer Lochtransferschicht, einer Elektroneninjektionsschicht, einer Elektronentransferschicht, einer Elektronenblockierschicht und einer Lochblockierschicht.

5. Organische lichtemittierende Vorrichtung nach Anspruch 2, wobei die organische Materialschicht ferner eine Verbindung der folgenden chemischen Formel 2 umfasst: 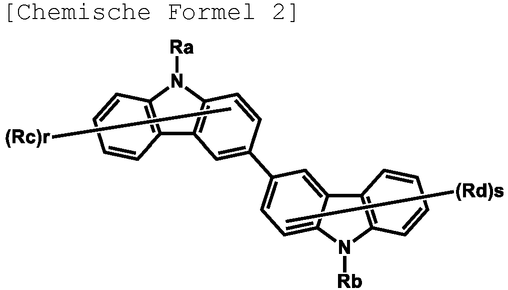 wobei in der chemischen Formel 2
Ra und Rb gleich oder verschieden voneinander sind und jeweils unabhängig eine substituierte oder unsubstituierte C6 - bis C60 -Arylgruppe oder eine substituierte oder unsubstituierte C2 - bis C60 -Heteroarylgruppe sind;
Rc und Rd gleich oder verschieden voneinander sind und jeweils unabhängig ausgewählt sind aus der Gruppe bestehend aus Wasserstoff; Deuterium; Halogen; einer Cyanogruppe; einer substituierten oder unsubstituierten C1 - bis C60 -Alkylgruppe; einer substituierten oder unsubstituierten C2 - bis C60 - Alkenylgruppe; einer substituierten oder unsubstituierten C2 - bis C60 -Alkinylgruppe; einer substituierten oder unsubstituierten C1 - bis C60 -Alkoxygruppe; einer substituierten oder unsubstituierten C3 - bis C60 - Cycloalkylgruppe; einer substituierten oder unsubstituierten C2 - bis C60 -Heterocycloalkylgruppe; einer substituierten oder unsubstituierten C6 - bis C60 -Arylgruppe; einer substituierten oder unsubstituierten C2 - bis C60 -Heteroarylgruppe und einer substituierten oder unsubstituierten Amingruppe; und
r und s eine ganze Zahl von 0 bis 7 sind.

6. Organische lichtemittierende Vorrichtung nach Anspruch 5, wobei die chemische Formel 2 durch eine der folgenden Verbindungen dargestellt ist:

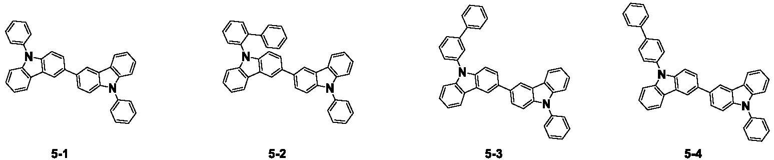

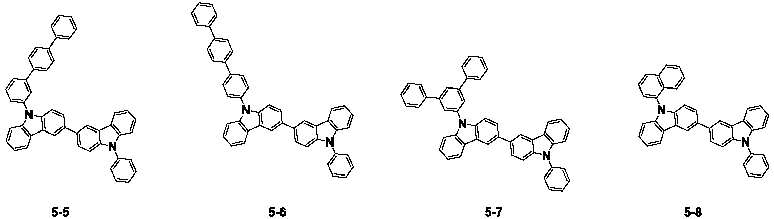

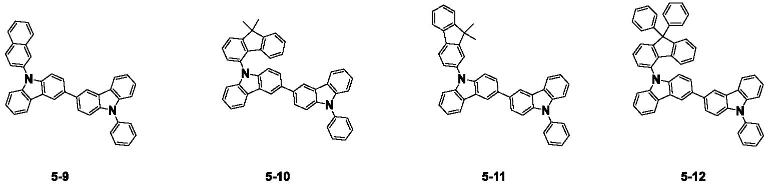

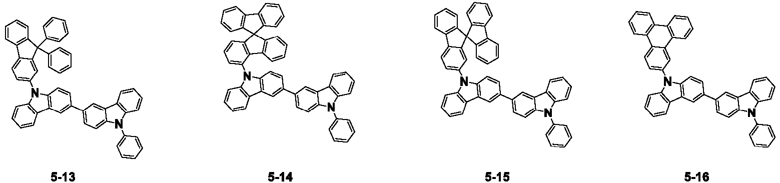

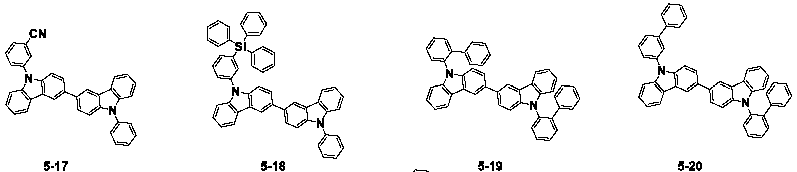

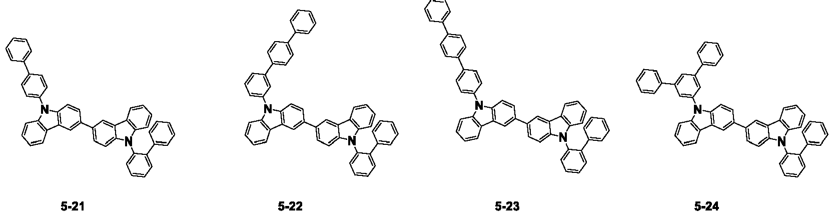

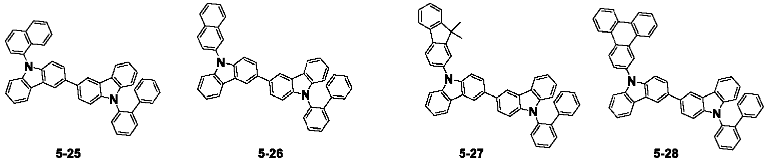

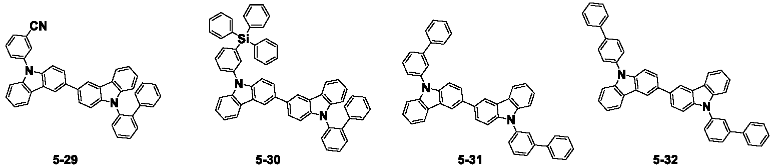

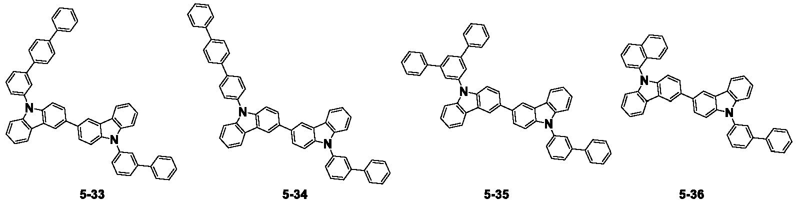

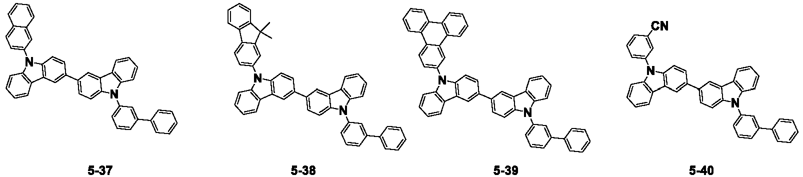

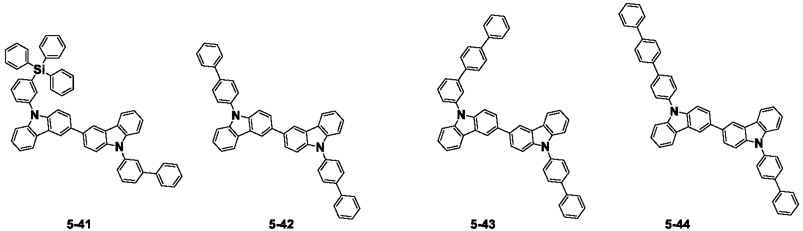

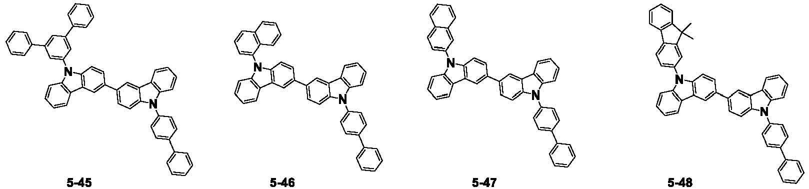

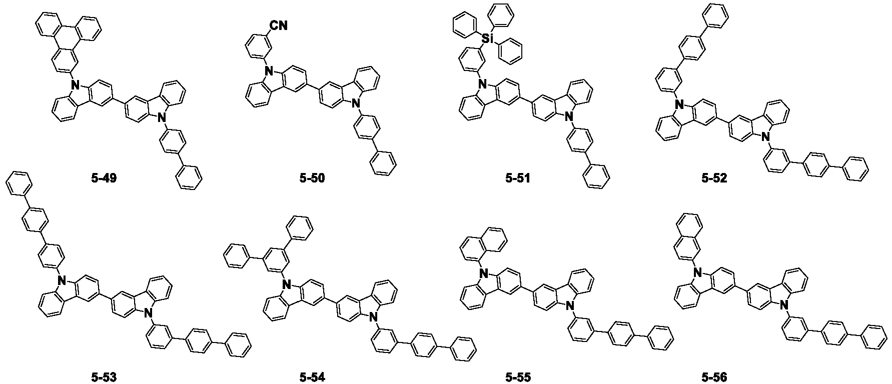

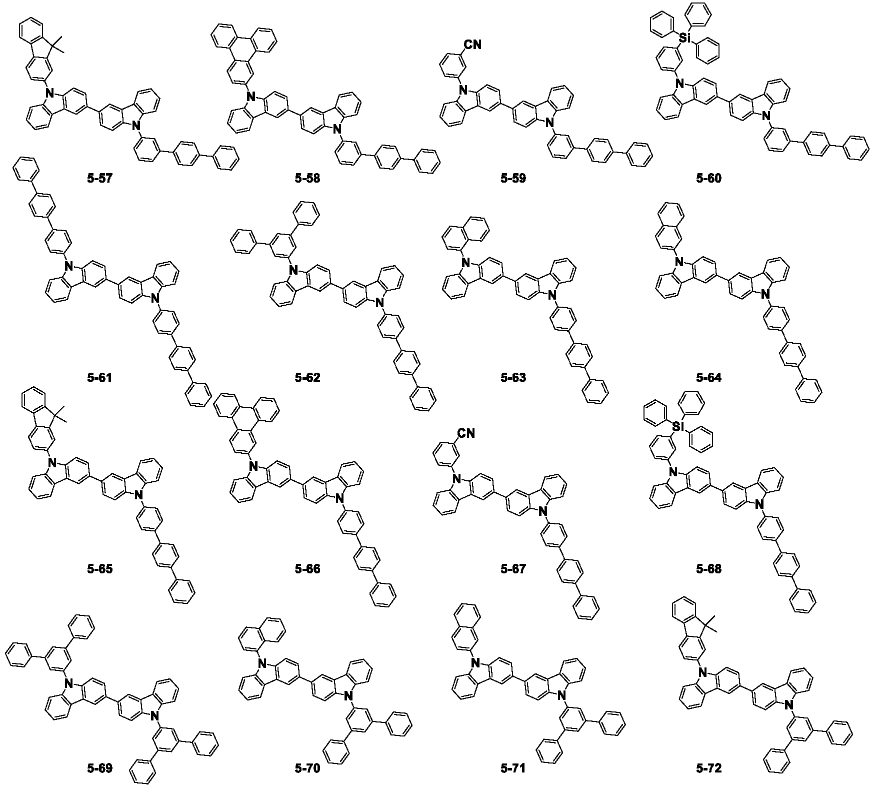

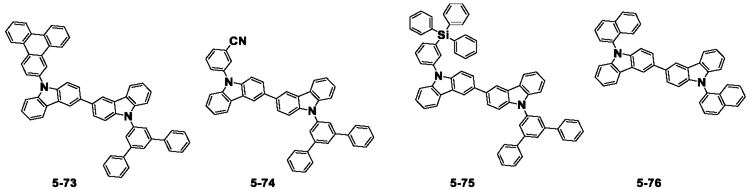

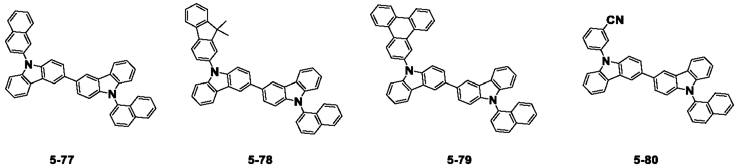

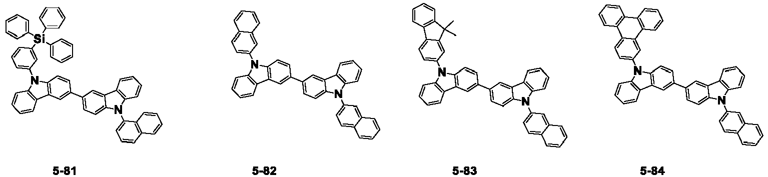

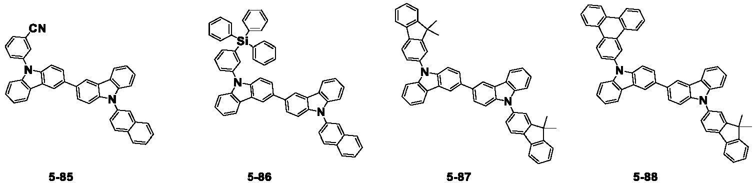

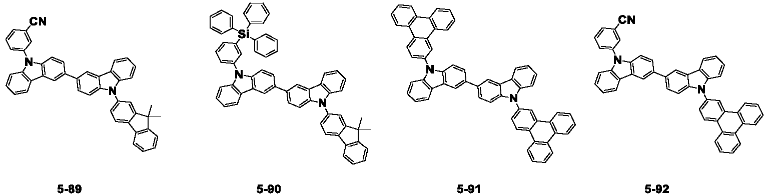

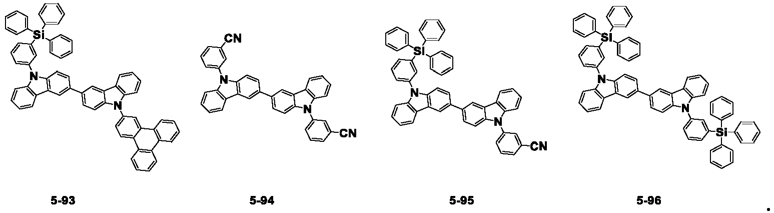

## Revendications

1. Composé hétérocyclique représenté par l'un quelconque des composés suivants :

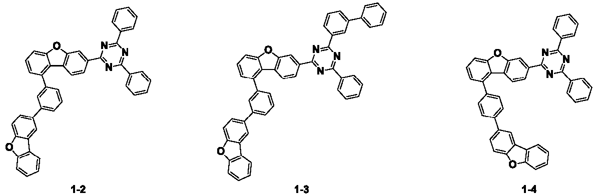

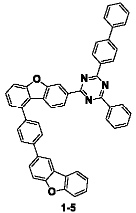

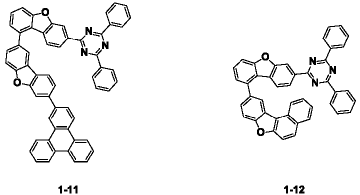

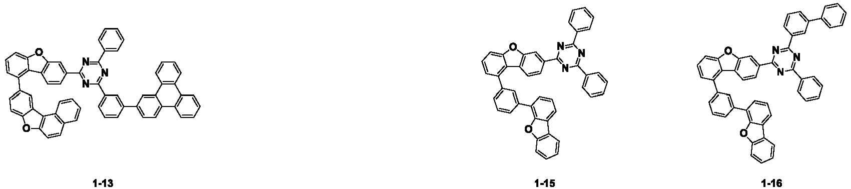

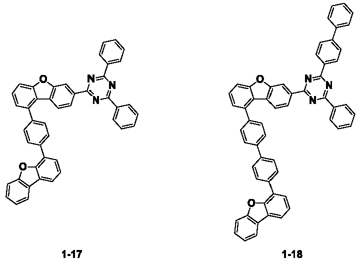

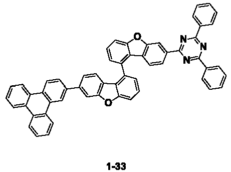

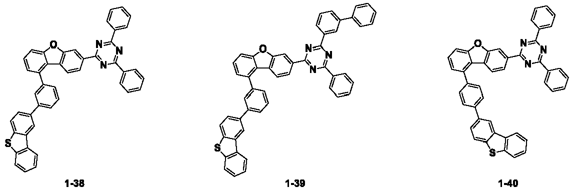

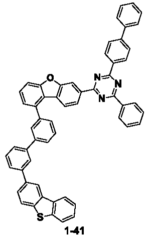

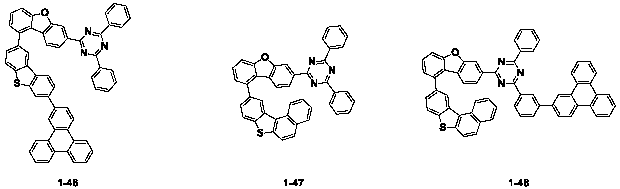

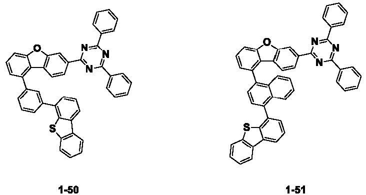

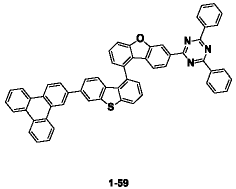

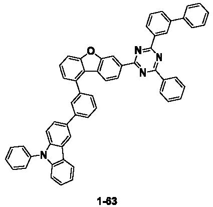

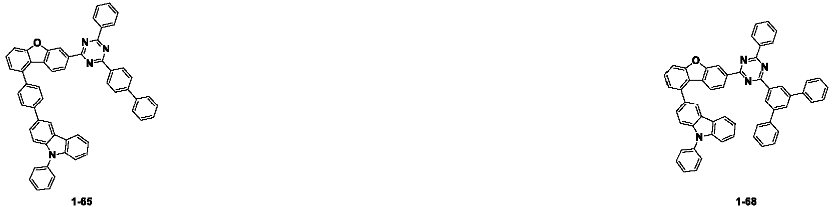

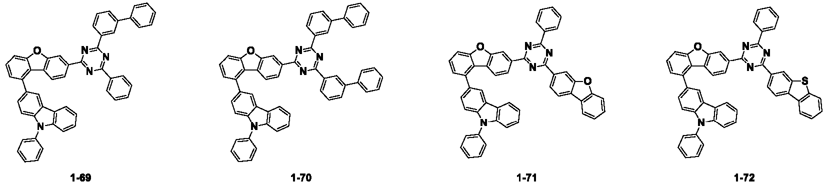

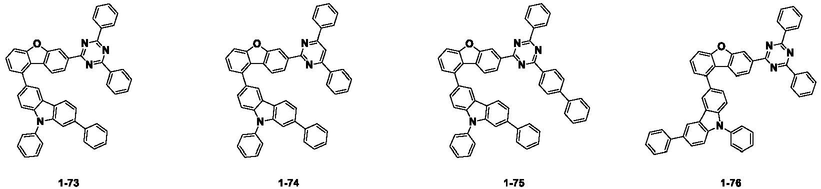

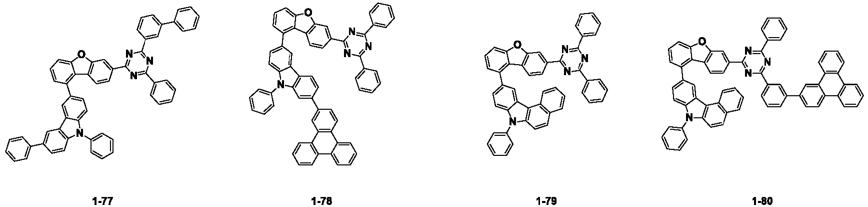

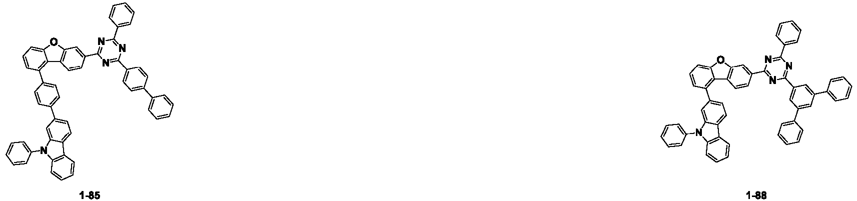

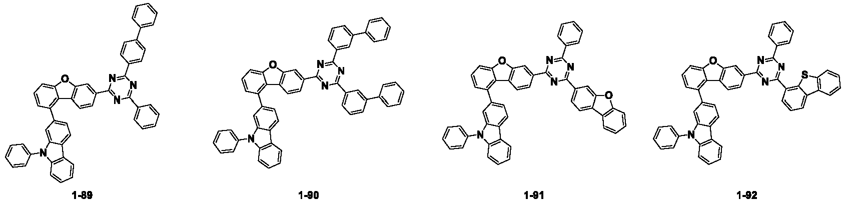

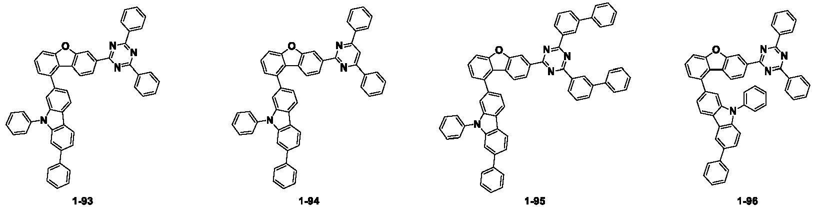

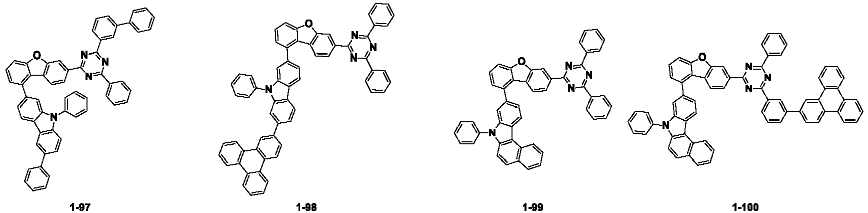

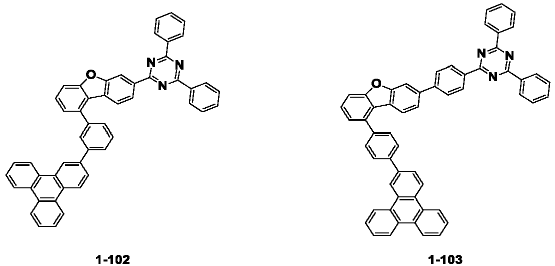

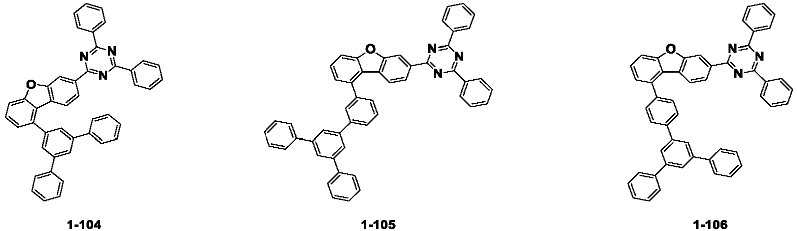

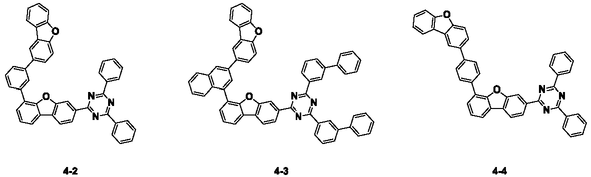

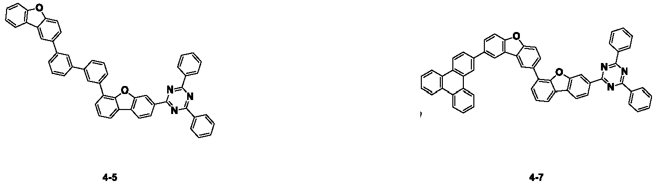

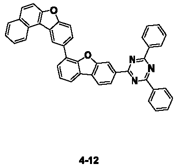

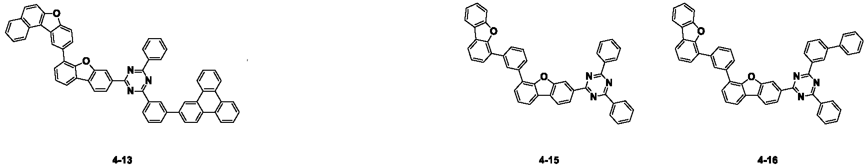

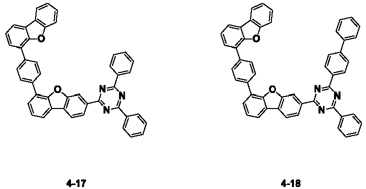

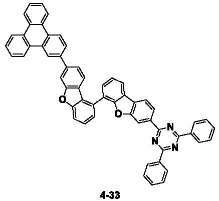

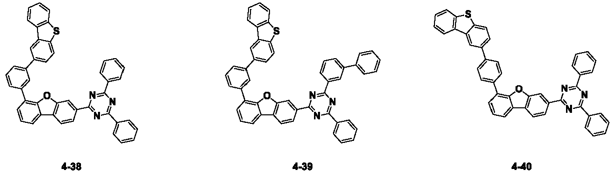

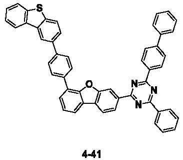

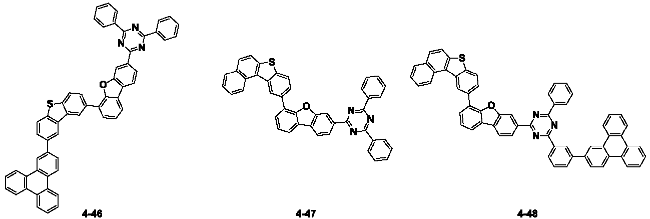

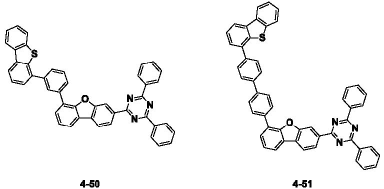

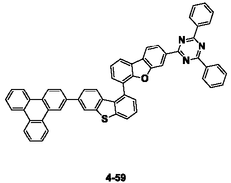

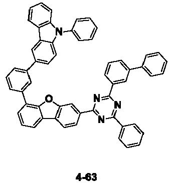

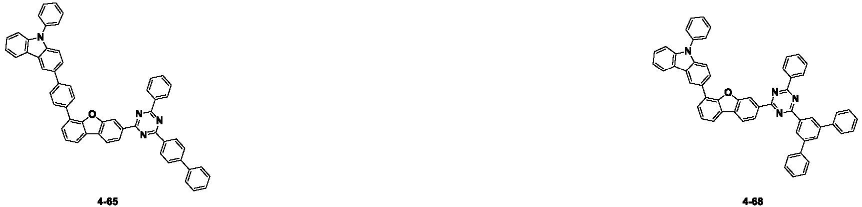

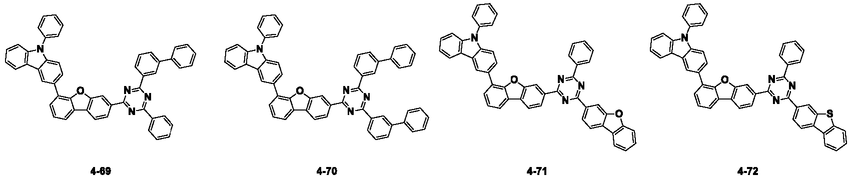

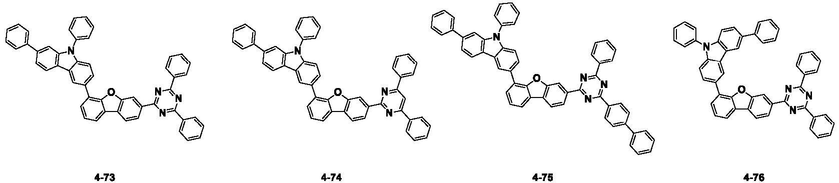

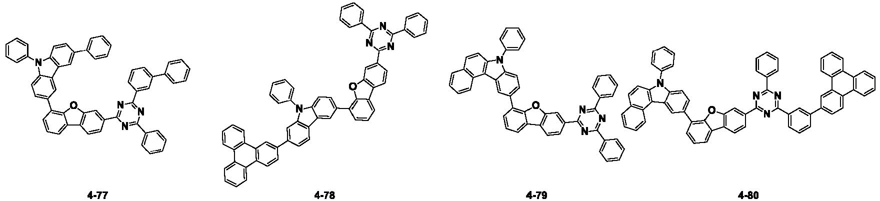

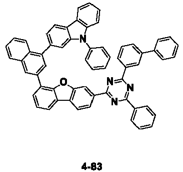

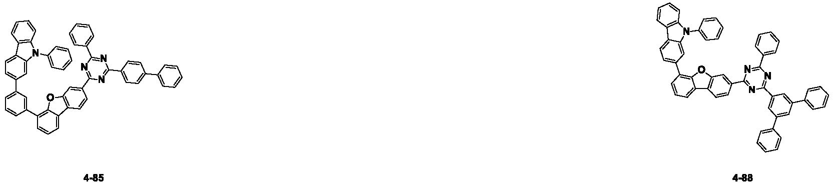

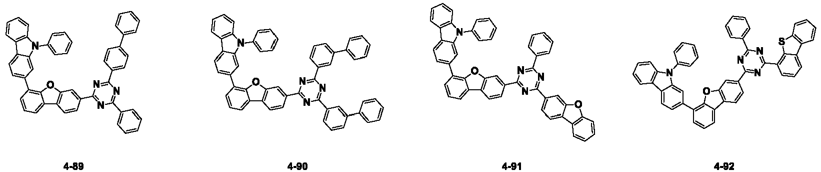

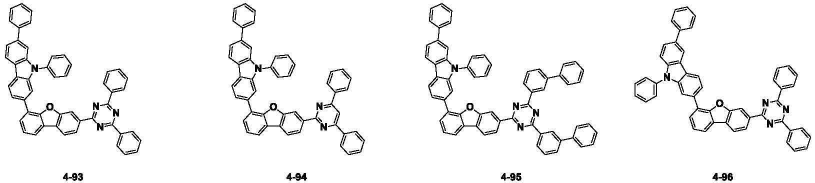

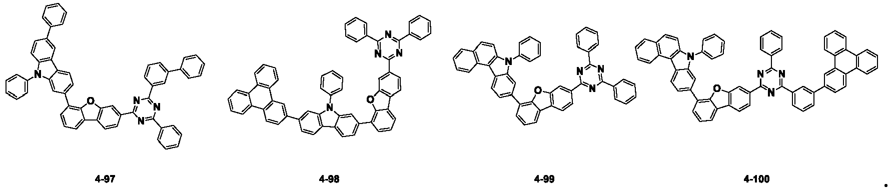

2. Dispositif électroluminescent organique comprenant :
une première électrode ;
une seconde électrode disposée à l'opposé de la première électrode ; et
une ou plusieurs couches de matériau organique disposées entre la première électrode et la seconde électrode,
dans lequel une ou plusieurs couches des couches de matériau organique comprennent le composé hétérocyclique de la revendication 1.

3. Dispositif électroluminescent organique de la revendication 2, dans lequel la couche de matériau organique comprend une couche électroluminescente, une couche d'injection d'électrons, une couche de transfert d'électrons, une couche de blocage d'électrons ou une couche de blocage de trous et la couche électroluminescente, une couche d'injection d'électrons, une couche de transfert d'électrons, une couche de blocage d'électrons ou une couche de blocage de trous comprend le composé hétérocyclique.

4. Dispositif électroluminescent organique de la revendication 2, comprenant en outre une, deux ou plusieurs couches choisies dans le groupe constitué d'une couche électroluminescente, une couche d'injection de trous, une couche de transfert de trous, une couche d'injection d'électrons, une couche de transfert d'électrons, une couche de blocage d'électrons et une couche de blocage de trous.

5. Dispositif électroluminescent organique de la revendication 2, dans lequel la couche de matériau organique comprend en outre un composé de la formule chimique 2 suivante : 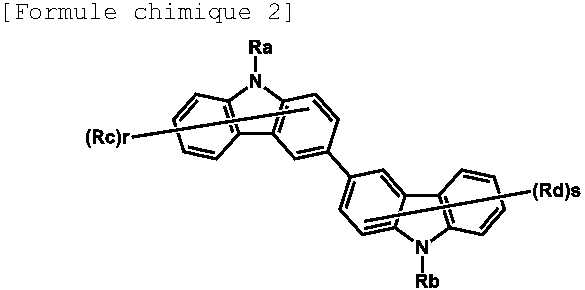 dans la formule chimique 2,
Ra et Rb sont identiques ou différents l'un de l'autre, et chacun indépendamment un groupe aryle en C6 à C60 substitué ou non substitué ; ou un groupe hétéroaryle en C2 à C60 substitué ou non substitué ;
Rc et Rd sont identiques ou différents l'un de l'autre, et chacun indépendamment choisi dans le groupe constitué d'hydrogène ; deutérium ; halogène ; un groupe cyano ; un groupe alkyle en C1 à C60 substitué ou non substitué ; un groupe alcényle en C2 à C60 substitué ou non substitué ; un groupe alcynyle en C2 à C60 substitué ou non substitué ; un groupe alcoxy en C1 à C60 substitué ou non substitué ; un groupe cycloalkyle en C3 à C60 substitué ou non substitué ; un groupe hétérocycloalkyle en C2 à C60 substitué ou non substitué ; un groupe aryle en C6 à C60 substitué ou non substitué ; un groupe hétéroaryle en C2 à C60 substitué ou non substitué ; et un groupe amine substitué ou non substitué ; et
r et s sont un entier de 0 à 7.

6. Dispositif électroluminescent organique de la revendication 5, dans lequel la formule chimique 2 est représentée par l'un quelconque des composés suivants :
